(19) Europäisches Patentamt / European Patent Office / Office européen des brevets

(11) **EP 1 924 295 B1**

(12) **EUROPEAN PATENT SPECIFICATION**

(45) Date of publication and mention
of the grant of the patent:
**06.06.2012 Bulletin 2012/23**

(21) Application number: **06809321.0**

(22) Date of filing: **15.09.2006**

(51) Int Cl.:
**A61L 15/22** (2006.01)   **C08J 3/24** (2006.01)

(86) International application number:
**PCT/IB2006/053324**

(87) International publication number:
**WO 2007/031969 (22.03.2007 Gazette 2007/12)**

(54) **AN ABSORBENT MEMBER COMPRISING A WATER ABSORBING AGENT**

SAUGFÄHIGES GLIED UMFASSEND EINEN WASSERAUFNEHMENDEN WIRKSTOFF

ELEMENT ABSORBANT COMPRENANT UN AGENT ABSORBANT L'EAU

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.09.2005 JP 2005270765**

(43) Date of publication of application:
**28.05.2008 Bulletin 2008/22**

(73) Proprietor: **The Procter & Gamble Company Cincinnati, OH 45202 (US)**

(72) Inventors:
• **IWAMURA, Taku**
  **Himeji-shi, Hyogo (JP)**
• **MITSUKAMI, Yoshiro**
  **Himeji-shi, Hyogo (JP)**

(74) Representative: **Kremer, Véronique Marie Joséphine**
  **Procter & Gamble Service GmbH**
  **Sulzbacher Strasse 40-50**
  **65824 Schwalbach am Taunus (DE)**

(56) References cited:
**EP-A- 1 504 772**    **EP-A2- 0 287 970**
**WO-A-2004/069915**    **US-A1- 2005 137 546**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Technical Field

This invention relates to an absorbent member for use in absorbent articles, especially diapers and training pants. The absorbent member comprises an absorbing agent made by a method comprising the steps of mixing a water absorbing resin, water and a mixing aid without adding a water-soluble radical polymerization initiator and an ethylenically unsaturated monomer, and irradiating the resultant mixture with ultraviolet rays while keeping the mixture still agitating the mixture in order to maintain a mixed state. Incidentally, the term "water absorbing agent" as used in this invention is referred to as a product resulting from modifying (cross-linking) the surface of a water absorbing resin.

Background Art

The water absorbent resin has been hitherto used as one component for hygienic materials such as sanitary cotton, disposable diaper, and absorbents for other kinds of body fluid. As concrete examples of the water absorbent resin, hydrolyzate of starch-acrylonitrile graft polymer, neutralized starch-acrylic acid graft polymer, saponified vinyl acetate-acrylic acid ester copolymer, hydrolyzate of acrylonitrile copolymer or acrylamide copolymer, and the product of cross-linkage thereof, and partially neutralized cross-linked acrylic acid may be cited. These water absorbent resins invariably possess an internal cross-linked structure and exhibit no solubility in water.

The characteristic properties which these water absorbent resins are expected to possess include high absorption capacity against no pressure and against pressure, excellent absorption speed, high gel strength, and fully satisfactory suction force necessary for sucking water from a medium, for example. Since the water absorbing properties are affected by crosslink density, they do not necessarily manifest positive correlations with one another as evinced by the fact that an increase in the crosslink density leads to an increase in the gel strength but a decrease in the amount of water absorbed. Particularly, the absorption capacity is in a contradictory relation with the absorption speed, the gel strength, and the suction force, for example. The water absorbent resin which has acquired an enhanced absorption capacity, therefore, possibly shuns uniform absorption of water and forms portions of partial aggregation of itself when the water absorbent resin particles contact with water and induces extreme degradation of the absorption speed because the water is not diffused throughout the entire volumes of water absorbent resin particles.

For the purpose of relaxing this phenomenon and obtaining a water absorbent resin which has a high absorption capacity and a comparatively satisfactory absorption speed, a method for giving the water absorbent resin particles a surface coated with a surfactant or a nonvolatile hydrocarbon has been available. This method indeed exalts the dispersibility of the initially absorbed water but brings no sufficient effects in enhancing the absorption speed and the suction force of the individual resin particles.

As a means to produce a polyacrylic acid type polymer of high water absorbing property, a method which comprises causing an aqueous composition having a partial alkali metal salt of polyacrylic acid as a main component and having a low crosslink density to be heated in the presence of a water-soluble peroxide radical initiating agent thereby introducing a crosslink therein by radical cross-linkage has been proposed (U. S. Patent No. 4910250). It is difficult to distribute uniformly internal cross-links in the polymer and uneasy to adjust the crosslink density. Thus, a measure of preparing a polymer which contains water-soluble polyacrylic acid gel having low crosslink density and then heating the polymer together with a persulfate added thereto as a polymerization initiator is adopted. Patent Document 1 claims to realize precise control of crosslink density by adjusting the amount of the initializing agent to be added and, owing to the uniform presence of crosslink in the polymer, acquire perfect water absorbing properties and obtain as well a water absorbent resin devoid of stickiness.

While the persulfate which is used in the Patent Document 1 mentioned above is decomposed by heat, it is decomposed by ultraviolet rays and generates radicals (J. Phys. Chem., 1975, 79, 2693, and J. Photochem. Photobiol., A, 1988, 44, 243). Since the persulfate fulfills a function as a polymerization initiator, the aqueous solution of a water-soluble vinyl monomer, when exposed to radiation, undergoes polymerization and radical cross-linkage simultaneously and produces a hydrogel (JP-A-2004-99789). A reaction system which forms an internal crosslink by adding a hydrophilic polymer component, a photo-polymerization initiator, and a cross-linking agent together and irradiating them with ultraviolet rays has been known (WO 2004/031253).

Meanwhile, a method which gives a water absorbent resin a surface treatment with a cross-linking agent and imparts thereto a surface of a heightened crosslink density has been also known (U. S. Patent No. 4666983 and U. S. Patent No. 5422405, for example). Such water absorbent resins as cited in the preceding patent documents entail the presence of a reactive functional group on their surfaces. By effecting introduction of a crosslink between functional groups in consequence of the addition of a surface cross-linking agent capable of reacting with the functional groups, it is made possible to give to the water absorbent resin a surface of increased crosslink density and enable the water absorbent resin to acquire water absorbing properties perfect even under pressure.

Further, since the use of the surface cross-linking agent mentioned above requires the reaction for the formation of cross-links to be performed at a high temperature for a long time and entails the problem of suffering persistence of the cross-linking agent in the unaltered state, a method which, by causing an aqueous solution containing a peroxide radical initiating agent to contact a resin and heating the resin, accomplishes introduction of cross-links into polymer molecular chains in the neighborhood of the surface of the resin by virtue of decomposition of the radical initiating agent has been proposed (U. S. Patent No. 4783510). In a working example of this method, a water absorbent resin exhibiting an exalted absorption capacity was obtained by affecting the heating with superheated steam at 130°C for 6 minutes.

Further, JP-A-2005-97585 discloses a technique for modifying the surface of the water absorbing resin and enhancing the absorbency against pressure by adding a processing liquid containing a radical polymerizing compound and a particulate water absorbing resin and irradiating the resultant mixture with active energy rays. Since this method uses a radical polymerizing compound, however, it has greatly lowered the absorbency against no pressure and entailed a very high cost.

JP-A-63-260907 discloses a technique for decreasing residual monomer content in a water absorbing resin by irradiating a water absorbing resin having specific water content with ultraviolet rays without adding a radical polymerizing compound. However, this technique does not involve the flow of the water absorbing resin during the irradiation with ultraviolet rays, therefore the modification of the surface of the water absorbing resin was performed extremely unevenly, if any.

Disclosure of the Invention

The object of introducing a surface cross-link to a water absorbing resin consists in producing a water absorbing resin excelling in the balance between absorbency and absorption speed. Generally, a cross-linking agent possessing at least two functional groups capable of reacting with the functional group existing in the surface of a water absorbing resin is required to react with the water absorbing resin. The cross-linking agents which fulfill this requirement include polyhydric alcohols, polyvalent glycidyl ethers, haloepoxy compounds, polyvalent aldehydes, polyvalent amines, and polyvalent metal salts, for example. Since these cross-linking agents generally are deficient in reactivity, they require the relevant reactions to be performed at elevated temperatures and occasionally placed under application of heat for a long time. Thus, the reactions call for enormous energy and time.

Even in the method for surface treatment disclosed in U.S. Patent No. 4,783,510, which uses a peroxide radical initiator as a cross-linking agent, the efficient reaction process needs a high temperature and the additional improvement of productivity is also demanded.

Then, the method disclosed in JP-A-2005-97585 suffers a great decrease of the absorbency against no pressure, as well as incurs a high cost owing to the use of a radical polymerizing compound.

Further, the method disclosed in JP-A-1988-260907 is desirable economically in terms that a radical polymerizing compound is not used. However, since the method disclosed in JP-A-1988-260907 does not involve the flow of the water absorbing resin during the irradiation with ultraviolet rays, the modification of a surface of a water absorbing resin is performed extremely unevenly and thus the resultant water absorbing resin has high possibility not to manifest excellent water absorption properties.

In the present state of affairs described above, this invention is aimed at providing an absorbent member for use in absorbent articles, especially for use in diapers and training pants. The absorbent member comprises an absorbing agent made by a method for producing a water absorbing agent excelling in such water absorption properties as absorbency and absorbing speed against no pressure or against pressure at a low cost with a high efficiency of production.

The present inventors have made a diligent study in search of a method for producing a water absorbing agent resulting from modifying the surface of a water absorbing resin and has consequently discovered that the uniform modification of the surface of a water absorbing resin can be attained by mixing the water absorbing resin and a water in the presence of a mixing aid and irradiating the resultant mixture with ultraviolet rays while keeping the mixture in a mixed state. This invention has been perfected as a result.

Moreover, the method, whereby the water absorbing agent comprised by the absorbent member of this invention is made, enables a modified surface without either using a surface cross-linking agent which has been an essential component for the conventional method or performing a treatment at a high temperature for a long time and permits the production of a target water absorbing agent exhibiting an exalted absorbency against pressure and excelling in such water absorbing properties as absorbency against pressure at a low cost with a high efficiency of production.

It has been simultaneously found that the water absorbing agent repress decrease of the absorbency against no pressure and excels particularly in the balance of water absorbing properties.

Heretofore, the surface cross-linkage which used a reaction by heating has necessitated a treatment at an elevated temperature as in the range of 100 to 300°C, depending on the kind of a surface cross-linking agent to be incorporated. The method whereby the water absorbing agent comprised by the absorbent member of this invention is made, permits the surface-modification solely by irradiation with ultraviolet rays and does not always necessitate heat.

Furthermore this method can reduce the energy costs required for the production because this invention can shorten the time of the treatment to a large extent compared with the conventional method.

Since the method, whereby the water absorbing agent comprised by the absorbent member of this invention is made, does not use any water-soluble radical polymerization initiator or ethylenically unsaturated monomer which is considered to be activated with ultraviolet rays to induce the formation of surface cross-linkage as well as any surface cross-linking agent reacting by heat, the raw material cost therefor can be decreased.

In respect the material properties, since it does not use any surface cross-linking agent, it can improve only the absorbency of the produced water absorbing agent against pressure considerably without any decrease in the absorbency under no pressure.

Also, the method, whereby the water absorbing agent comprised by the absorbent member is made of this invention is made, can repress the formation of aggregates which are easily occurred at the time of mixing the water absorbing resin and water in the presence of a mixing aid and permit the uniform surface-modification by irradiating the resultant mixture with ultraviolet rays while keeping the mixture in a mixed state.

The other objects, characteristics, and advantages of this invention will be clarified by taking into consideration the following explanation and the preferred modes of embodiment illustrated in the drawings attached hereto.

Brief Description of the Drawings

Fig. 1 is a schematic diagram of a device used for determining the absorbency 0.3 psi against pressure.
Fig. 2 is a schematic cross section of a separable flask made of quartz and used in the working examples.
Fig. 3 (A) is a perspective view of stirring vanes used in the working examples and Fig. 3 (B) is a side view of the stirring vanes used in the working examples.

Detailed Description of the Embodiment

This invention refers to an absorbent member for use in absorbent articles, especially for use in diaper or training pants, comprising an absorbing agent made by a method comprising

a) a step of mixing a water absorbing resin, water, and a mixing acid without adding a water-soluble radical polymerization initiator and an ethylenically unsaturated monomer and
b) a step of irradiating the resultant mixture with an ultraviolet rays while still agitating the mixture in order to maintain a mixed state..

(a) Water absorbing resin

The water absorbing resin usable in this invention is a water-swelling water-insoluble cross-linked polymer capable of forming a hydrogel. The term "water-swelling" as used in this invention refers to the water absorbency against no pressure in an aqueous 0.9 weight% sodium chloride solution (physiological saline water) such that a given sample absorbs essentially at a ratio of not less than 2 g/g, preferably at a ratio in the range of 5 to 100 g/g and more preferably at a ratio in the range of 10 to 60 g/g. The term "water-insoluble" refers to the eluted soluble uncross-linked content (the water-soluble polymer compound) in the water absorbing resin which preferably is in the range of 0 to 50 weight%, more preferably not more than 25 weight%, still more preferably not more than 15 weight%, and particularly preferably not more than 10 weight%. Incidentally, the numerical values of the water absorbency against no pressure and the eluted soluble content are those found by the method of determination specified in the working example to be cited herein below.

This invention preferably makes use of a water absorbing resin which possesses a cross-linked structure obtained by polymerizing an acid group-containing unsaturated monomer from the viewpoint of liquid absorbing properties. Incidentally, this invention regards as an acid group-containing unsaturated monomer such a monomer as acrylonitrile which is converted by hydrolysis subsequent to polymerization into an acid group after the polymerization. Preferably, an acid group-containing unsaturated monomer which contains an acid group at the time of polymerization is used.

The water absorbent resin which can be used in this invention does not need to be particularly restricted but is only required to be capable of being obtained by polymerizing a monomer component essentially containing an ethylenically unsaturated monomer by means of any of the known methods.

The ethylenically unsaturated monomer is not particularly restricted but is preferred to be a monomer possessing an unsaturated double bond at the terminal thereof. Examples of the monomer according to this invention are anionic monomers such as (meth)acrylic acid, 2-(meth)acryloyl ethane sulfonic acid, 2-(meth)acryloyl propane sulfonic acid, 2-(meth)acrylamide-2-methyl propane sulfonic acid, vinyl sulfonic acid, and styrene sulfonic acid and salts thereof; nonionic hydrophilic group-containing monomers such as (meth)acrylamide, N-substituted (meth)acrylamide, 2-hydroxyethyl (meth)acrylate, and 2-hydroxypropyl(meth)acrylate; and amino group-containing unsaturated monomers such as N,N-dimethylaminoethyl (meth)acrylate, N,N-diethylaminoethyl (meth)acrylate, N,N-diethylaminopropyl (meth)acrylate, and N,N-dimethylaminopropyl (meth)acrylamide and quaternized products thereof. These monomers may be used either singly or in the form of a mixture of two or more members. Among monomers enumerated above, (meth)acrylic acid, 2-(meth)acryloyl ethane sulfonic acid, 2-(meth)acrylamide-2-methylpropane sulfonic acid, and salts thereof, N,N-dimethylaminoethyl(meth)acrylate and quaternized N,N-dimethylaminoethyl (meth)acrylate, and (meth)acrylamide prove preferable and acrylic acid and/or a salt thereof are particularly preferable.

When an acrylic acid salt is used as the monomer, the monovalent salt of acrylic acid selected from among alkali metal salts, ammonium salt, and amine salt of acrylic acid proves favorable from the viewpoint of the ability of the water absorbent resin to absorb water. More preferably, the alkali metal salt of acrylic acid and particularly preferably the acrylic acid salt selected from among sodium salt, lithium salt, and potassium salt prove favorable.

In the production of the water absorbent resin, other monomer components than the monomers enumerated above may be used in amount not impairing the effect of this invention. Eexamples of such other monomer components are hydrophobic monomers such as aromatic ethylenically unsaturated monomers having carbon numbers in the range of 8 to 30, aliphatic ethylenically unsaturated monomers having carbon numbers in the range of 2 to 20, alicyclic ethylenically unsaturated monomers having carbon numbers in the range of 5 to 15, and alkyl esters of (meth)acrylic acid containing alkyl groups having carbon numbers in the range of 4 to 50. The proportion of such a hydrophobic monomer is generally in the range of 0 to 20 weight parts based on 100 weight parts of the ethylenically unsaturated monomer mentioned above. If the proportion of the hydrophobic monomer exceeds 20 weight parts, this will possibly degrade the water absorbing property of the produced water absorbent resin.

The water absorbent resin which is used in this invention is insolubilized by the formation of an internal crosslink. This internal crosslink may be the product obtained by the self-cross-linkage using no cross-linking agent. It may be formed by using an internal cross-linking agent possessing not less than two polymerizable unsaturated group and/or not less than two reactive functional groups in the molecular unit.

The internal cross-linking agent of this description does not need to be particularly restricted. Examples of the inner cross-linking agent are N,N'-methylenebis(meth)acrylamide, N-niethylol (meth)acrylamide, glycidyl (meth)acrylate, (poly)ethylene glycol di(meth)acrylat.e, (poly)propylene glycol di(meth)acrylate, glycerin tri(meth)acrylate, glycerin acrylate methacrylate, polyvalent metal salts of (meth)acrylic acid, trimethylol propane tri(meth)acrylate, triallyl amine, triallyl cyanurate, triallyl isocyanurate, triallyl phosphate, ethylene glycol diglycidyl ether, (poly)glycerol glycidyl ether, and polyethylene glycol diglycidyl ether. These internal crosslinking agents may be used in the form of a mixture of two or more members.

The amount of the internal cross-linking agent to be used is preferably in the range of 0.0001 to 1 mol%, more preferably in the range of 0.001 to 0.5 mol%, and still more preferably in the range of 0.005 to 0.2 mol%. If this amount is below 0.0001 mol%, this will possibly prevent the internal cross-linking agent from being introduced into the resin. Conversely, if the amount exceeds 1 mol%, this will possibly unduly heighten the gel strength of the water absorbent resin and lower the absorption capacity. For the introduction of the cross-linked structure into the interior of the polymer by the use of the internal cross-linking agent, it suffices to add the internal cross-linking agent into the reaction system before, during, or after the polymerization of the monomer or after neutralization of the produced polymer.

For the purpose of producing the water absorbent resin, it suffices to polymerize the monomer components including the monomer mentioned above and the internal cross-linking agent in an aqueous solution thereof. The polymerization initiators which can be used in this case are water-soluble radical polymerization initiators including persulfates such as potassium persulfate, ammonium persulfate, and sodium persulfate; potassium peracetate, sodium peracetate, potassium percarbonate, sodium percarbonaate, and t-butyl hydroperoxide; hydrogen peroxide; azo compounds such as 2,2'-azobis(2-amidinopropane)-dihydrochloride and photopolymerization initiators including 2-hydroxy-2-methyl-lphenyl-propan-1-on, for example. The water-soluble radical polymerization initiators mentioned above may be combined with a reducing agent such as a sulfite, L-ascorbic acid, or a ferric salt so as to be used as redox type initiators.

The concentration of the monomer in the aqueous monomer solution mentioned above does not need to be particularly restricted but falls preferably in the range of 15 to 90 wt. % and more preferably in the range of 35 to 80 wt. %. If this concentration is below 15 wt. %, this may be disadvantageous with regard to heat consumption and with regard to the time required for drying because the resultant hydrogel may have an unduly large water content.

The method to be adopted for the polymerization is not particularly restricted but may be selected from among the known methods such as solution polymerization, reversed-phase suspension polymerization, precipitation polymerization, and bulk polymerization. Among these methods, the aqueous solution polymerization which comprises dissolving a monomer in an aqueous solution and polymerizing it in the aqueous solution, and the reversed phase suspension polymerization prove particularly advantageous on account of the ease of control of a polymerization reaction and the performance of a produced water absorbent resin.

In initiating the aforementioned polymerization, the polymerization initiator mentioned above is used to effect this initiation. Besides the polymerization initiator mentioned above, active energy rays as ultraviolet rays, electron radiation, and rays may be used either singly or in combination with a polymerization initiator. Though the temperature in initiating the polymerization depends on the kind of polymerization initiator to be used, it falls preferably in the range of 15 to 130°C and more preferably in the range of 20 to 120°C. If the temperature in initiating the polymerization deviates from the range mentioned above, this may be disadvantageous with regard to increased residual monomer in the produced water absorbent resin and the self cross-linking reaction may proceed too long, consequently degrading the water absorbing property of the water absorbent resin.

The term "reversed phase suspension polymerization" refers to a method of polymerization performed on an aqueous monomer solution suspended in a hydrophobic organic solvent. It is disclosed in U.S. Patent No. 4093776, No. 4367323, No. 4446261, No. 4683274, and No. 5244735, for example. The term "aqueous solution polymerization" refers to a method for polymerizing an aqueous monomer solution without using a dispersing solvent. It is disclosed in U.S. Patent No. 4625001, No. 4873299, No. 4286082, No. 4973632, No. 4985518, No. 5124416, No. 5250640, No. 5264495, No. 5145906, and No. 5380808 and European Patent No. 0811636, No. 0955086, and No. 0922717, for example. The monomers and the initiators which are cited by way of illustration in these methods of polymerization can be applied to this invention.

The aqueous solution polymerization can be implemented by polymerizing a partially neutralized acrylic acid or polymerizing an acid group-containing monomer such as e.g. acrylic acid and thereafter neutralizing the resultant polymer with an alkali compound such as sodium hydroxide, ammonium hydroxide, sodium carbonate, or ammonium carbonate.

Preferably, the neutralization degree of the produced water absorbing resin containing an acid group (the mol% of the neutralized acid group in the whole acid group) is in the range of not less than 50 mol% and less than 95 mol%, preferably in the range of 53 to 85 mol%, still more preferably in the range of 55 to 75 mol%, and most preferably in the range of 60 to 65 mol%. If the aforementioned neutralization degree is below 50 mol%, this will possibly lower the water absorbency of the water absorbing resin. If it exceeds 95 mol%, this will possibly inhibit or even prevent the surface treatment from proceeding.

After polymerization, generally the cross-linked polymer is in the form of a hydrogel. While this invention allows this hydrogel-like cross-linked polymer to be unaltered in order to form the water absorbing resin, it prefers the polymer to be dried until a water content (%) (100 - solid content (%)) is achieved, which will be specifically described below.

The water absorbent resin which is used in this invention is preferably a powdery water absorbent resin which is obtained by polymerizing a monomer having acrylic acid (salt) particularly as its main component. The hydrogel-like cross-linked polymer which is obtained by polymerization is preferably dried and subsequently pulverized to a particulate water absorbent resin. The drying may be effected by using a drier such as a hot air drier at a temperature in the range of 100 to 220°C and more preferably in the range of 120 to 200°C.

For use in the pulverization, among shear primary crushers, impact shredders, and high speed rotary grinders included in the powdering machines classified in Table 1.10 of the Particle Technology Handbook (first edition, compiled by Particle Technology Association), the powdering machines which possess at least one of the powdering mechanisms such as cutting, shearing, striking, and rubbing can be adopted particularly favorably. Among the powdering machines, the powdering machines which have cutting and shearing as main mechanisms can be used particularly advantageously. A roll mill (roll rotary type) powdering machine may be cited as a preferred example.

The water absorbing resin to be used in this invention is preferably in a powdered form. Preferably it is a powdered water absorbing resin which contains particles of diameters of not less than 150 $\mu$m and less than 850 $\mu$m (as defined by sieve classification) at a ratio in the range of 90 weight% to 100 weight%, preferably in the range of 93 weight% to 100 weight%, particularly preferably in the range of 95 weight% to 100 weight%, still more preferably in the range of 98 weight% to 100 weight%, and most preferably in the range of 99 weight% to 100 weight%.

When the aforementioned powdered water absorbing resin happens to have mainly particles of at least 850 $\mu$m in diameter and the water absorbing agent manufactured from this resin is used in a disposable diaper, the disposable diaper may give an unpleasant touch to the skin and may possibly rupture the topsheet of the diaper. Also, the water absorption speed may be reduced. Using mainly particles of less than 150 $\mu$m in diameter, the resin powder may aggregate upon exposure to water, and may also fail to undergo uniform irradiation with the active energy rays. Further, this may allow low or even no enhancement of water absorption capacity against pressure. When the resin having a particle size of less than 150 $\mu$m is used in a disposable diaper, for example, of the particles may pass through the topsheet of the diaper onto the outer surface of the diaper.

The weight average particle diameter (D50) of the water absorbing resin is preferably not less than 300 $\mu$m and less than 500 $\mu$m, more preferably in the range of 300 to 450 m, and particularly preferably in the range of 300 to 400 $\mu$m.

The logarithmic standard deviation of particle size distribution ($\sigma\zeta$) of the water absorbing resin is preferably in the range of 0.20 to 0.45, more preferably in the range of 0.20 to 0.40, particularly desirably in the range of 0.20 to 0.35, and still more preferably in the range of 0.20 to 0.30. The logarithmic standard deviation of particle size distribution (σζ) means a magnitude which decreases with decreasing particle size distribution. For the water absorbing resin of this invention, it is desirable to have a particle size distribution which is not simply narrow but is widened to a certain extent. If the logarithmic standard deviation of particle size distribution (σζ) of the aforementioned water absorbing resin exceeds 0.45, this will possibly have a negative impact on the water absorbency against pressure because of an unduly wide particle size distribution. If it is below 0.20, this will possibly conspicuously lower the productivity of the water absorbing resin.

Incidentally, the weight average particle diameter of the water absorbing resin (D50) and the logarithmic standard deviation of particle size distribution (σζ) of the water absorbing resin are the magnitudes which are determined by the method specified in the working example which will be cited herein below.

The water absorbing resin to be used in this invention has an eluted soluble content which is preferably not less than 0.1 weight parts and less than 30 weight parts, more preferably in the range of 1 to 25 weight parts, still more preferably in the range of 3 to 20 weight parts, and most preferably in the range of 5 to 15 weight parts per 100 weight parts of the aforementioned water absorbing resin. If the eluted soluble content of the water absorbing resin is below 0.1 weight parts, this will possibly necessitate a large amount of an inner cross-linking agent during the course of polymerization, heighten the cost, and further lower the water absorbency. Conversely, if the eluted soluble content of the water absorbing resin exceeds 30 weight parts, this will possibly induce liquation of a soluble component during the addition of water or an aqueous solution, inhibiting the soluble component from functioning as a binder between the adjacent water absorbing resin particles. Also, this may give rise to lumps of powder, prevent the surface treatment from proceeding uniformly, and also prevent the water absorbency against pressure from being sufficiently exalted.

The water absorbing resin to be used in this invention may also be obtained by preparing a precursor water absorbing resin having a relatively low neutralization degree and then mixing the precursor water absorbing resin with a base. Heretofore, polyfunctional surface treating agents have been used for the surface treatment (surface cross-linking treatment). These polyfunctional surface treating agents can react with the carboxyl groups (-COOH) in the water absorbing resin but fail to react with the salt thereof (such as, for example, -COONa). Thus, by polymerizing an ethylenically unsaturated monomer mixture (the mixture of acrylic acid and sodium acrylate, for example) adjusted in advance so as to have a suitable -COOH/-COONa ratio in order to produce a water absorbing resin having -COOH and -COONa distributed uniformly therein, and using the produced water absorbing resin for the surface treatment with polyfunctional surface treating agents, it is possible to obtain uniform cross-linkage. A water absorbing resin may be obtained by polymerizing an acid type ethylenically unsaturated monomer as acrylic acid such as e.g. a main component and then the resultant polymer is neutralized with an alkali compound such as e.g. sodium hydroxide or sodium carbonate. If this water absorbing resin is surface cross-linked with polyfunctional surface treating agents, the cross-linkage is indeed at an advantage in yielding a small eluted soluble content of the water absorbing resin. Nevertheless, this cross-linkage inevitably results in a decrease of the water absorption property because -COOH and -COONa are not uniformly distributed. Thus, it has not been advisable to subject the water absorbing resin obtained by the latter method to the conventional surface cross-linkage with the polyfunctional surface treating agents. According to the method, whereby the water absorbing agent comprised by the absorbent member of this invention is made, it is possible to modify a water absorbing resin obtained by polymerizing a monomer/monomer mixture having as main component such an acid type ethylenically unsaturated monomer as acrylic acid, thereby obtaining a precursor water absorbing resin having a low neutralizing degree. Thus precursor water absorbing resin is neutralized with an alkali compound such as sodium hydroxide or sodium carbonate, whereby a water absorbing resin is obtained which has -COOH and -COONa distributed uniformly thereon. The water absorbing agent which is obtained by this method can manifest excellent water absorption properties.

A precursor water absorbing resin having a low neutralization degree as used herein refers to a precursor water absorbing resin having a neutralization degree (the mol% of the neutralized acid group in all the acid groups) in the range of 0 to 50 mol% and preferably in the approximate range of 0 to 25mol%. The precursor water absorbing resin having such a low neutralization degree can be obtained by following the aforementioned method while using a monomer mixture including such an acid group-containing monomer as acrylic acid preferably in such a manner as to acquire the aforementioned neutralizing degree. Thus, the detailed explanation of the precursor will be omitted here.

The water content of the water absorbing resin to be used for the method of producing a water absorbing agent according to this invention does not need to be particularly restricted so long as the water absorbing resin possesses fluidity. The water content of the water absorbing resin after being dried at 180°C for three hours is in the range of 0 to 20 weight%, preferably in the range of 0 to 10 weight%, and more preferably in the range of 0 to 5 weight%. Incidentally, the water content of the water absorbing resin is the magnitude determined by the method specified in the working example which will be cited herein below.

Further, in the method of this invention, during the course of mixing of the water absorbing resin and water mentioned above, other additives may be additionally used with the objective to impart other functions to the water absorbing agent unless they exert such adverse effects as deterioration on the water absorbing resin obtained in consequence of the radiation of the ultraviolet rays. Anti-fungus agents, aromatic agents, deodorants, etc. may be cited as concrete examples of the additives mentioned above.

(b) Mixture of water absorbing resin, water, and mixing aid

In the method, whereby the water absorbing agent comprised by the absorbent member of this invention is made, the water-soluble radical polymerization initiator and the ethylenically unsaturated monomer are not added at the time of mixing the water absorbing resin, water, and the mixing aid. Therefore, method, whereby the water absorbing agent comprised by the absorbent member of this invention is made, does not include the case of adding only the water-soluble radical polymerization initiator or adding only the ethylenically unsaturated monomer at the time of mixing the water absorbing resin, water.

As regards the mixing ratio of the aforementioned water absorbing resin and water, water is added in an amount preferably of not less than 1 weight part and less than 50 weight parts, more preferably in the range of 2 to 30 weight parts, still more preferably in the range of 3 to 15 weight parts, particularly preferably in the range of 4 to 13 weight parts, and most preferably in the range of 5 to 10 weight parts, based on 100 weight parts of the water absorbing resin. If the amount of water is below 1 weight part, this will possibly prevent radiation with ultraviolet rays from inducing surface cross-linkage as expected. If it is not less than 50 weight parts, this will possibly lower the water absorbency against no pressure and against pressure of the produced water absorbing resin.

In this invention, the mixing aid is not particularly limited, so long as that it should be a water-soluble or water-dispersible compound except an ethylenically unsaturated monomer or a water-soluble radical polymerization initiator, and as long as it can repress the agglomeration of the water absorbent resin with water and improve the mixing of the aqueous solution with the water absorbent resin. Since the addition of the mixing aid can repress the agglomeration of the water absorbent resin with water, and induce the uniform mixing of the aqueous solution with the water absorbent resin, the ultraviolet rays, when irradiation is done in the subsequent step, can equally and evenly irradiate the water absorbent resin and thus the uniform surface cross-linkage of the entire water absorbent resin can be achieved. To be specific, a surfactant, a water-soluble polymer compound, a hydrophilic organic solvent, a water-soluble inorganic compound, an inorganic acid, an organic acid, and an organic salt are available. Herein, the term "water-soluble compound" means a compound having solubility in 100 g of water at room temperature of not less than 1 g, preferably not less than 10 g.

When the mixing aid is used, the mode of using the mixing aid is not particularly restricted. Though the mixing aid may be used in the form of powder or be dissolved, dispersed, or suspended in a solution, it is preferably used in the form of an aqueous solution. Incidentally, the aqueous solution may incorporate other solvents than water within the range in which the solubility of the mixing aid is not impaired.

When the mixing aid is used, the point of time for adding the mixing aid is not particularly restricted. The mixing aid may either be added before adding the water absorbing resin and water thereto, subsequently mixing them altogether or the mixing aid may be added simultaneously with the water absorbing resin, water.

The aqueous solution containing the mixing aid has a surface tension preferably in the range of 0.0004 - 0.00075 N/cm (40 to 75 dynes/cm), more preferably in the range of 0.00045 - 0.0007 N/cm (45 to 70 dyes/cm) still more preferably in the range of 0.00048 - 0.00065 N/cm (48 to 65 dynes/cm), and most preferably in the range of 0.0005 - 0.0006 N/cm (50 to 60 dynes/cm). If the surface tension is below 0.0004 N/cm (40 dynes/cm), this will possibly lower the capillary capacity of the water absorbing resin during the course of absorbing water and, when the resin is used as in a disposable diaper, increase the amount of the absorbed liquid remaining on or flowing back onto on the topsheet of the diaper. If the Surface tension exceeds 0.00075 N/cm (75 dynes/cm), this will possibly disrupt the uniformity of the mixture of the water absorbing resin and the aqueous solution and prevent the ultraviolet rays from radiating uniformly. The surface tension of the aqueous solution containing the aforementioned mixing aid is the magnitude which is determined by the method specified in the working example cited herein below.

Regarding the surfactant used as the mixing aid, at least one surfactant selected from the group consisting of nonionic surfactants and anionic surfactants having an HLB of not less than 7 can be used. Specific examples of the surfactant are sorbitan fatty acid esters, polyoxyethylene sorbitan fatty acid esters, polyglycerin fatty acid esters, polyoxyethylene alkyl ethers, polyoxyethylene alkyl phenol ethers, polyoxyethylene acyl esters, sucrose fatty acid esters, higher alcohol sulfuric acid esters, alkyl naphthalene sulfonic acid salts, alkyl polyoxyethylene sulfate salts, and dialkyl sulfosuccinic acid salts. Polyoxyethylene alkyl ethers are particularly advantageous as surfactants. The molecular weight of the polyoxyethylene alkyl ether is preferably 200 to 100,000 and more preferably 500 to 10,000. If the molecular weight is unduly large, this may lower the solubility of the surfactant in water, allowing no addition of the amount to be added, increase the viscosity of the solution, and impair the mixing property of the surfactant with the water absorbing resin. Conversely, if the molecular weight is unduly small, this may degrade the effect as a mixing aid.

As preferred examples of the water-soluble polymer compound, macromolecules possessing solubility of not less than 1 wt. % in water at room temperature may be cited, for example, polyvinyl alcohol, polyethylene oxide, polyethylene glycol, polypropylene glycol, polyacryl amide, polyacrylic acid, sodium polyacrylate, polyethylene imine, methyl cellulose, carboxymethyl cellulose, hydroxy-ethyl cellulose, hydroxypropyl cellulose, dextrin, sodium alginate, and starch. Polyethylene glycol proves particularly advantageous as water-soluble polymer compound. The molecular weight of the compound is preferably 200 to 100,000 and more preferably 500 to 10,000, similarly to the polyoxyethylene alkyl ether.

Examples of the hydrophilic organic solvent are alcohols such as methyl alcohol, ethyl alcohol, propyl alcohol, isopropyl alcohol, butyl alcohol, isobutyl alcohol, and t-butyl alcohol; ketones such as methylethyl ketone; ethers such as dioxane, alkoxy (poly)ethylene glycol, and tetrahydrofuran; amides such as g-caprolactam and N,N-dimethyl formamide; sulfoxides such as dimethyl sulfoxide; and polyhydric alcohols such as ethylene glycol, diethylene glycol, propylene glycol, triethylene glycol, tetraethylene glycol, 1,3-propane diol, dipropylene glycol, 2,2,4-trimethyl-1,3-pentadiol, glycerin, 2-butene-1,4-diol, 1,3-butane diol, 1,4-butane diol, 1,5-pentane diol, 1,6-hexane diol, 1,4-cyclohexane dimethanol, 1,2-cyclo-hexenol, trimethylol propane, diethanol amine, triethanol amine, polyoxypropylene, pentaerythritol, and sorbitol. These solvents may be used either singly or in the form of a mixture of two or more members.

As preferred examples of the water-soluble inorganic compound, inorganic compounds possessing solubility of not less than 5 wt. % in water may be cited, for example, water-soluble metal salts which are salts of metals having valences of at least one. More specific examples of the water-soluble metal salt are alkali metal salts such as sodium chloride, sodium hydrogen sulfate, and sodium sulfate, ammonium salts such as ammonium chloride, ammonium hydrogen sulfate, and ammonium sulfate, alkali metal hydroxides such as sodium hydroxide and potassium hydroxide, polyvalent metal salts such as aluminum chloride, aluminum polychloride, aluminum sulfate, potassium alum, calcium chloride, calcium sulfate, calcium carbonate, magnesium chloride, magnesium sulfate, magnesium carbonate, zirconium sulfate, zirconium nitrate, zirconium acetate, zirconium carbonate, zirconium ammonium acetate, zirconium ammonium carbonate, zirconium oxychloride, zirconium chloride, titanium chloride, and titanium sulfate, and irreducible alkali metal salt pH buffer agents such as hydrogen carbonates, dihydrogen phosphates, and hydrogen phosphates.

Typical examples for inorganic acids salts are the salts of the inorganic acid selected from the group consisting of hydrochloride acid, sulfuric acid, phosphoric acid, carbonic acid, and boric acid may be cited such as alkali metal salts and alkaline earth metal salts. Then, as examples for organic acids salts, the salts of the organic acids selected from the group consisting of acetic acid, propionic acid, lactic acid, citric acid, succinic acid, malic acid, and tartaric acid, such as alkali metal salts and alkaline earth metal salts.

Among other compounds enumerated above, polyoxyethylene alkyl ethers, polyethylene glycol, and water-soluble metal salts are advantageously usable as a mixing aid.

When a macromolecular mixing aid such as a polyoxyethylene alkyl ether or polyethylene glycol is used, the number average molecular weight (Mn) of this mixing aid is preferably 100 to 500000, more preferably 200 to 100000, still more preferably 500 to 10000, and particularly preferably 1000 to 5000. If the number average molecular weight mentioned above is below 100, this will possibly impair the mixability of the water absorbing resin and water, induce formation of lumps of powder, prevent the surface cross-linkage from proceeding uniformly, and inhibit the acquisition of a sufficient water absorbency against pressure. If it exceeds 500000, this will possibly lower the solubility of the mixing aid in water and compel the production in an actual device to incur troubles.

These mixing auxiliaries may be used either singly or in the form of a mixture of two or more members. Then, the amount of the mixing aid to be added is not particularly limited but is only required to repress the aggregation of the water absorbing resin with water, and improve the mixability of water and the water absorbing resin. Specifically, this amount is preferably not less than 0.01 weight part and less than 50 weight parts, more preferably in the range of 0.03 to 20 weight parts, particularly preferably in the range of 0.05 to 10 weight parts, still more preferably in the range of 0.1 to 5 weight parts, and most preferably in the range of 0.3 to 1 weight parts per 100 weight parts of the water absorbing resin. Otherwise, in this invention, the mixing aid may be used preferably in the range of 0 to 40 weight%, more preferably in the range of 0.01 to 30 weight%, and still more preferably in the range of 0.1 to 10 weight%, based on the total amount of the aqueous solution.

Incidentally, as means to mix the water absorbing resin, water or an aqueous solution, and the mixing aid, a method which consists in effecting the mixture by the use of an ordinary mixing device such as, for example, V-shaped mixer, a ribbon type mixer, a screw type mixer, a rotary disc type mixer, an air current type mixer, a batch type kneader, a continuous kneader, a paddle type mixer, or a spade type mixer may be cited.

(c) Keeping the mixture in a mixed state

The method, whereby the water absorbing agent comprised by the absorbent member of this invention is made, comprises mixing the water absorbing resin, water and the mixing aid as described above and irradiating the resultant mixture with ultraviolet rays while keeping the mixture in a moving stream. Thereby, it is possible to perform the surface treatment uniformly and conspicuously exalting the water absorbency against pressure without decreasing the water absorbency against no pressure. The ultraviolet rays used in this process preferably have a wavelength of above 200nm and not more than 400nm.

For the purpose of fluidizing the mixture during the irradiation of the mixture with the ultraviolet rays, blowing a gas such as air, oxygen, or nitrogen into the fixture and fluidizing the aforementioned mixture are equally possible besides stirring the mixture.

When the mixture containing the water absorbing resin and water is fluidized by the motion of stirring, the stirring speed is preferably 300 to 1000 rpm, more preferably 400 to 750 rpm, and still more preferably 450 to 550 rpm. If the stirring speed is below 300 rpm, this will possibly prevent the ultraviolet rays from irradiating uniformly. If it exceeds 1000 rpm, this will possibly expose the mixture to mechanical damage and increase the content of fine particles in the water absorbing agent after irradiation with the ultraviolet rays.

The fluidization of the mixture of the water absorbing resin and water or an aqueous solution and the mixing aid may be implemented by using the heretofore known device. Examples of the known devices are a shaking type mixer, a shaking feeder, a ribbon type mixer, a conical ribbon type mixer, a screw type mixing and extruding device, an air current type mixer, a batch type kneader, a continuous type kneader, a paddle type mixer, a high speed fluid type mixer, and a free ascent flow type mixer.

(d) Irradiation with ultraviolet rays, the ultraviolet rays preferably having a wavelength of above 200nm and not more than 400nm

As a method for modifying the surface of a water absorbent resin (particles), the formation of a surface crosslinkage attained by using a surface crosslinking agent and promoting the relevant reaction by application of heat has been known to the public. For surface cross-linking the water absorbing resin as described above, compounds such as a polyhidric alcohol, a polyvalent glycidyl ether, a haloepoxy compound, or a polyvalent aldehyde which possesses a plurality of functional groups in the molecular unit thereof are used. Generally, when the polymerizing monomer component are heated to 100 to 300°C, these functional groups react with the carboxyl groups being present in the surface of the water absorbing resin and consequently induce formation of a cross-linked structure in the surface of the water absorbing resin.

Also, a method for modifying the surface of a water absorbent resin (particles) by adding a treatment liquid containing the radical polymerization compound to the water absorbing resin and irradiating the resultant mixture with active energy rays has been reported.

The method, whereby the water absorbing agent comprised by the absorbent member of this invention is made, however, is characterized by being capable of forming a cross-linked structure in the surface of a water absorbing resin by irradiating a mixture of the water absorbing resin, water and the mixing aid with active energy rays even in the absence of such a surfactant and a polymerizing monomer as mentioned above. Also by this characteristic property, it is made possible to exalt the water absorbency of the modified water absorbing agent against pressure (AAP) without lowering the water absorbency thereof against no pressure (CRC).

The ultraviolet rays preferably have a wavelength of above 200nm and not more than 400nm. The ultraviolet rays having a wavelength of above 200nm and not more than 350nm are more preferably used, and the ultraviolet rays having a wavelength of above 200nm and not more than 300nm are still more preferably used. If active energy rays with a wavelength exceeding 400 nm are used, irradiation will be deficient in energy and will possibly prevent the crosslinkage of the water absorbing resin from proceeding. If the active energy rays of 200 nm or below are used, irradiation will likely simultaneously induce cross-linkage and severance and possibly deteriorate the water absorbing resin.

As regards the conditions of the radiation, the intensity of radiation is in the range of 3 to 100 mW/cm$^2$ and the dosage of radiation is in the range of 100 to 10000 mJ/cm$^2$ when the ultraviolet rays are used. A high-pressure mercury lamp, a reduced-pressure mercury lamp, a metal halide lamp, and a halogen lamp are examples for suitable radiation sources. As long as the ultraviolet rays have a wavelength of preferably above 200nm and not more than 400nm, more preferably above 200nm and not more than 350nm, and still more preferably above 200nm and not more than 300nm, the source may additionally emit other radiant rays and wavelengths. The way to obtain this radiation is not particularly restricted. Incidentally, when the electron radiation is used, preferably the accelerating voltage is 50 to 800 kV and the absorbed dose rage is 0.1 to 100 Mrad.

Generally, the irradiation time is preferably not less than 0.1 minute and less than 60 minutes, more preferably not less than 1 minute and less than 30 minutes, still more preferably not less than 2 minutes and less than 20 minutes, and particularly preferably not less than 3 minutes and less than 15 minutes. For the fixed cross-link density, the time for the surface cross-linking treatment may be curtailed as when it exceeds 60 minutes in the case of using the conventional surface cross-linking agent If the time of radiation is below 0.1 minute, this will possibly prevent the surface treatment of the water absorbing resin from being sufficiently carried out. If it exceeds 60 minutes, this will possibly degrade the water absorbing resin due to the active energy rays.

The distance from the ultraviolet rays radiating lamp to the mixture containing the water absorbing resin, water, and the mixing aid is preferably 2 to 30 cm, more preferably 5 to 15 cm, and particularly preferably 8 to 15 cm. If this distance is below 2 cm, this will possibly result in adhesion of the mixture of the water absorbing rein and water or an aqueous solution onto the irradiation lamp. If the distance exceeds 30 cm, this will possibly not further increase the effect of the ultraviolet rays.

The surface treatment effected by irradiation with ultraviolet rays does not require any warming. It is, however, commendable to irradiate at elevated temperatures because this inhibits the risk of aggregation while mixing. It is only neccessary to elevate the temperature of the water absorbing resin to preferably less than 150°C, more preferably to less than 120°C, still more preferably only to between room temperature and 100°C, and particularly preferably to a temperature in the range of 50 to 100°C. Thus, the temperature can be set lower than the conventional surface treating temperature.

In the present invention, irradiation does not have to be carried out in an inert environment.

(e) Other treatments

After the irradiation of the active energy rays, the water absorbent resin may be optionally subjected to a heat treatment at a temperature in the range of 50 to 250°C for drying.

After irradiation with ultraviolet rays, the water absorbing resin may be a surface cross-linked by using any of the heretofore universally known surface cross-linking agents such as polyhydric alcohols, polyvalent epoxy compounds, and alkylene carbonates.

The method for producing the water absorbing agent according to this invention allows addition of a liquid passage enhancing agent to the water absorbing resin before, after, or during the radiation of the active energy rays. Examples of the liquid passage enhancing agents are mineral products such as talc, kaolin, fuller's earth, bentonite, activated clay, barite, natural asphaltum, strontium ore, ilmenite, and pearlite; aluminum compounds such as aluminum sulfate 14 - 18 hydrates (or anhydrides), potassium aluminum sulfate dodecahydrate, sodium aluminum sulfate dodecahydrate, aluminum chloride, aluminum polychloride, and aluminum oxide and aqueous solutions thereof; other polyvalent metal salts; hydrophilic amorphous silicas (examples, dry process: product of Tokuyama K.K. sold under the trademark designation of "Reolosil QS-20", precipitation process: products of Degussa Corp. sold under the trademark designations of "Sipernat 22S" and "Sipernat 2200"); and oxide complexes such as silicon oxide aluminum oxide magnesium oxide complex (example, product of Engelhard Corp sold under the trademark designation of "Attagel #50"), silicon oxide aluminum oxide complex, and silicon oxide magnesium oxide complex. These liquid passage enhancing agents are mixed preferably in an amount of 0 to 20 weight parts, more preferably in an amount of 0.01 to 10 weight part, and particularly preferably in an amount of 0.1 to 5 weight parts with 100 weight parts of the modified water absorbing resin. A water-soluble liquid passage enhancing agent is added in the form of an aqueous solution whereas a non water-soluble liquid passage enhancing agent is added in the form of powder or slurry. Other additives such as anti-fungus agent, deodorant, and chelating agent may be properly incorporated within the range mentioned above.

(f) Water absorbing agent

The term "water absorbing agent" as used in this invention refers to a water absorbing agent having a modified surface (i.e. a water absorbing agent which has undergone surface treatment).

When the method for producing a water absorbing agent according to this invention is executed, the produced water absorbing agent has an exalted absorbency against pressure without decreasing the absorbency against no pressure. It has been heretofore known that the formation of a surface cross-link greatly lowers the absorbency of physiological saline against no pressure and nevertheless exalts the capacity for retaining the absorbed liquid even in a state of applying pressure, namely the absorbency against pressure. According to the method of this invention, the absorbency of the water absorbing resin against pressure of 2.07 kPa is increased by at least 1 g/g even without using a water-soluble radical polymerization initiator and an ethylenically unsaturated monomer. This fact is thought to indicate that the method of this invention has introduced a surface cross-link to the surface of the water absorbing resin. In the solid state physical properties acquired after the modification, the absorbency under pressure is preferably not less than 3 g/g, more preferably not less than 5 g/g, still more preferably not less than 7 g/g, and particularly preferably not less than 10 g/g. Incidentally, the absorption capacity of the water absorbing resin against pressure of 2.07 kPa is the magnitude determined by the method specified in the working example cited herein below.

The water absorbing agent of this invention has an absorbency against pressure of 2.07 kPa of preferably not less than 15 g/g and less than 50 g/g, more preferably 18 to 40 g/g, still more preferably 20 to 35 g/g, and most preferably 25 to 30 g/g. If the absorbency against pressure mentioned above is below 15 g/g, this will possibly result in an water absorbing agent having an unduly small absorbency against pressure and, when the agent is used as in a disposable diaper, induce leakage of urine. If it exceeds 50 g/g, this will possibly increase the soluble component and induce gel blocking.

Then, the absorbency against no pressure (CRC) is preferably not less than 8 g/g, more preferably not less than 1.5 g/g, still more preferably not less than 20 g/g, and particularly preferably not less than 25 g/g. Though the upper limit of this absorbency is not particularly restricted, it is preferably not more than 50 g/g, more preferably not more than 40 g/g, and still more preferably not more than 35 g/g. If the absorbency against no pressure (CRC) is below 8 g/g, the water absorbing agent has an unduly small absorption capacity and is not suitable for use as an absorbent member in absorbent articles such as disposable diapers. If the absorbency against no pressure (CRC) exceeds 50 g/g, this will possibly weaken the gel strength and prevent the produced water absorbing agent from excelling in the absorbency against pressure.

Further, the water absorbing agent which is obtained according to this invention is characterized by having an extremely small residual monomer content. Since the water absorbing resin is used in absorbent articles such as disposable diapers, the smaller the residual monomer content is, the better the end use proves from the viewpoint of odor and safety. Ordinarily, the residual monomer content in the water absorbing resin is 200 to 500 ppm. The residual monomer content of the surface-treated water absorbing resin obtained by this invention is not more than 200 ppm (the lower limit is 0 ppm) in most cases. The residual monomer content of the modified water absorbing resin is preferably not more than 200 ppm, more preferably not more than 150 ppm, and still more preferably not more than 100 ppm.

Further, the water absorbing agent which is obtained by this invention has a small solid content compared to water absorbing agents obtained by conventional methods of modification wherein the water absorbing resin and surface cross-linking agents are subjected to elevated temperatures. This is because the method of production according to this invention does not require elevated temperatures for the reaction and, therefore, allows the water contained in the aqueous solution added to the water absorbing resin to remain substantially intact even after the reaction. When the water content in the water absorbing agent is large, this promotes the formation of fine particulates having a particle diameter of not more than 150 $\mu$m, which prevents the generation of static electricity on the surface or particulates, thereby which constituting blocking during the pneumatic transportation. This results in degradation of physical properties due to physical damage occuring during pneumatic transportation. The solid content of the water absorbing agent is preferably not more than 95%, more preferably not more than 93%, still more preferably not more than 90%, and particularly preferably not more than 85%. Though the lower limit of the solid content is not particularly restricted, a solid content below 70% possibly lowers the absorbency of the water absorbing agent per unit weight.

The form of the surface-treated water absorbing agent which is obtained by this invention can be properly adjusted by the conditions of treatment such as the form of the water absorbent resin before treatment and the agglomeration and molding of the treated water absorbent resin after the treatment. Generally, however, the modified water absorbent resin has a powdery form. This powder has a weight average particle diameter (specified by classification with sieves) which is in the range of 10 to 1,000 $\mu$m and preferably in the range of 200 to 600 $\mu$m. In this powder, the content of particles having diameters of 150 to 850 $\mu$m is preferably in the range of 90 to 100 % by weight and more preferably in the range of 95 to 100 % by weight based on the weight of the water absorbent resin.

The method for production according to this invention causes the fine particles generated during the production of the water absorbing resin to agglomerate during surface cross-linking. Thus, even when the water absorbing resin yet to be modified happens to contain fine particles, the method for producing the water absorbing agent according to this invention enables agglomeration of the fine particles and consequently decreases the amount of fine particles contained in the surface-treated water absorbing agent. The particle-size distribution of the produced water absorbing agent is shifted towards higher particle sizes compared to the particle size of the water absorbing agent yet to be modified. The ratio of this shifting, however, is varied by the amount of water to be mixed with the water absorbing resin, the condition of radiation with ultraviolet rays, and the mode of fluidizing the mixture during radiation.

The water absorbing agent to be obtained by the method of this invention have uniformly formed surface cross-links formed over the whole surface of the water absorbing resin with a high cross-link density. Thus, the water absorbing agents have the characteristic properties expected of a good water absorbing resin such as, for example, absorbency, absorption speed, gel strength, and suction force, at unusually high levels.

When this invention is executed in the presence of an ethylenically unsaturated monomer, the execution does not conform to the object of this invention because the absorption capacity against no pressure is decreased.

The water-soluble radical polymerization initiator is supposed to be dissolved in a ratio of not less than 10 weight% in water (25°C). Examples of this polymerization initiator are persulfates such as ammonium persulfate, sodium persulfate, and potassium persulfate; hydrogen peroxide; and water-soluble azo compounds such as 2,2'-azobis-2-amidinopropane dihydro-chloride and 2-2'-azobis[2-2(-imidazolin-2-yl)propane] dihydrochloride.

In accordance with this invention, the surface treatment of the water absorbent resin is effected fully satisfactorily even at a reaction temperature near room temperature and the surface-treated water absorbent agent consequently obtained is enabled to manifest at extremely high levels such characteristic properties as absorption capacity, absorption speed, gel strength, and suction force which the water absorbent resin is expected to possess. The water absorbent resin which is obtained by this invention, therefore, is optimally usable for sanitary cotton, disposable diapers, and other sanitary materials for absorbing body fluid.

**Absorbent articles**

The absorbent members made by the method of the present invention are preferably used as absorbent cores in absorbent articles. As used herein, absorbent article refers to devices that absorb and contain liquid, and more specifically, refers to devices that are placed against or in proximity to the body of the wearer to absorb and contain the various exudates discharged from the body. Absorbent articles include but are not limited to diapers, adult incontinent briefs, diaper holders and liners, sanitary napkins and the like.

Preferred absorbent articles of the present invention are diapers and training pants. As used herein, "diaper" and "training pants" refers to an absorbent article generally worn by infants and incontinent persons about the lower torso.

Absorbent articles especially suitable for the present invention typically comprise an outer covering including a liquid pervious topsheet, a liquid impervious backsheet and an absorbent core generally disposed between the topsheet and the backsheet. The absorbent core may comprise any absorbent material that is generally compressible, conformable, non-irritating to the wearer's skin, and capable of absorbing and retaining liquids such as urine and other certain body exudates. In addition to the SAP particles of the present invention, the absorbent core may comprise a wide variety of liquid-absorbent materials commonly used in disposable diapers and other absorbent articles such as comminuted wood pulp, which is generally referred to as air felt.

Exemplary absorbent structures for use as the absorbent assemblies are described in U.S. Patent No. 5,137,537 entitled "Absorbent Structure Containing Individualized, Polycarboxylic Acid Crosslinked Wood Pulp Cellulose Fibers" which issued to Herron et al. on August 11, 1992; U.S. Patent 5,147,345 entitled "High Efficiency Absorbent Articles For Incontinence Management" issued to Young et al. on September 15, 1992; U.S. Patent No. 5,342,338 entitled "Disposable Absorbent Article For Low-Viscosity Fecal Material" issued to Roe on August 30, 1994; U.S. Patent No. 5,260,345 entitled "Absorbent Foam Materials For Aqueous Body Fluids and Absorbent Articles Containing Such Materials" issued to DesMarais et al. on November 9, 1993; U.S. Patent No. 5,387,207 entitled "Thin-Until-Wet Absorbent Foam Materials For Aqueous Body Fluids And Process For Making Same" issued to Dyer et al. on February 7, 1995; U.S. Pat. No. 5,397,316 entitled "Slitted Absorbent Members For Aqueous Body Fluids Formed Of Expandable Absorbent Materials" issued to LaVon et al. on March 14, 1995; and U.S. Patent No. 5,625,222 entitled "Absorbent Foam Materials For Aqueous Fluids Made From high In al. on July 22, 1997.

EXAMPLES

Now, this invention will be explained more specifically below with reference to working examples and comparative examples. This invention is not limited to these working examples. Incidentally, the varying properties of water absorbing resins described therein were determined by the following methods. All the operations described therein were carried out under the conditions of room temperature (20 to 25°C) and humidity 50 RH% unless special conditions were mentioned.

(1) Centrifuge retention capacity (abbreviated as CRC)

The centrifuge retention capacity (CRC) indicates the absorbency exhibited by a given sample after 30 minutes' standing against no pressure in an aqueous 0.90 weight% saline solution. A pouch (85 mm x 60 mm) made of non-woven fabric (made by Nangoku Pulp Kogyo K.K. and sold under the trademark designation of "Heatlon Paper, Type GSP-22") was packed uniformly with 0.200 g of a given water absorbing resin or water absorbent agent, heat sealed, and immersed in a large excess (generally about 500 ml) of an aqueous 0.90 weight% sodium chloride solution at room temperature. The pouch was pulled up from the solution after 30 minutes of standing therein and drained for three minutes with the centrifugal force (250 G) described in edana ABSORBENCY 11 441.1-99 by using a centrifugal separator (made by Kokusansha K.K. and sold under the product code of "Type H-122"). Then, the drained pouch was weighed to find the weight W1 (g) thereof. The same procedure was repeated without using a water absorbing resin and the drained pouch was weighed to find the weight W0 (g). Then, the centrifuge retention capacity (CRC) (g/g) was calculated in accordance with the following formula using the found values of W1 and W0.

$$\text{Centrifuge retention capacity (CRC) (g/g)} = (W1\,(g) - W0\,(g))/(\text{weight (g) of water absorbing resin or water absorbing agent}) - 1$$

(2) Absorbency against pressure 0.3 psi (abbreviated as "AAP 0.3")

The absorbency against pressure 0.3 psi (AAP 0.3) indicates the absorbency indicated by a given sample after 60 minutes' standing under pressure of 2.07 kPa (0.3 psi) in an aqueous 0.90 weight% saline solution.

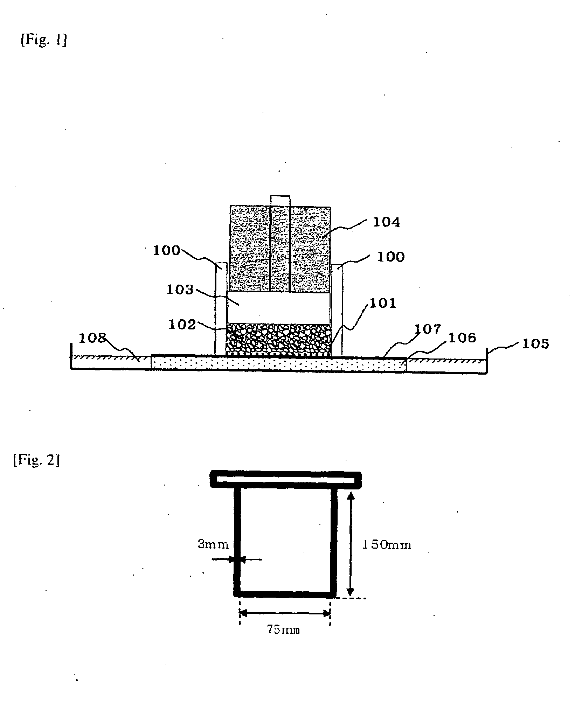

In an apparatus illustrated in Fig. 1, a 400-mesh metal gauze 101 (aperture 38 m) made of stainless steel was fused to the bottom of a plastic supporting cylinder 100 having an inside diameter of 60 mm. On the metal gauze, 0.900 g of a given water absorbing resin or water absorbing agent was uniformly scattered under the conditions of room temperature (20 to 25°C) and humidity 50 RH% and a piston 103 and a load 104 adjusted so as to exert a load of 2.07 kPa (0.3 psi) on the scattered sample, given an outside diameter slightly smaller than 60 mm and prevented from producing an interspace between themselves and the supporting cylinder, and enabled to produce an uninterrupted vertical motion were mounted on the scattered sample sequentially in the order mentioned. The whole of the apparatus was weighed to find the weight W2 (g) thereof.

Inside a petri dish 105 having a diameter of 150 mm, a glass filter 106 having a diameter of 90 mm (made by Sogo Rikagaku Glass Seisakusho K.K., having a pore diameter of 100 to 120 m) was placed and an aqueous 0.90 weight% saline solution 108 (20 to 25°C) was added till the same level as the upper surface. On the soaked glass filter in the petri dish, one filter paper 107 having a diameter of 90 mm (made by ADVANTEC Toyo K.K. and sold under the product name "JIS P 3801, No. 2," and having a thickness of 0.26 mm and a retention, particle diameter of 5 m) was placed so as to have the surface completely wetted, with the excess liquid removed therefrom.

The whole apparatus mentioned above was placed on the aforementioned filter paper and the water absorbing resin or the water absorbing agent was left absorbing the liquid under the load. After the lapse of one hour thence, the whole apparatus was lifted and weighed to find the weight W3 (g) thereof. The absorbency against pressure 0.3 psi (AAP 0.3) (g/g) was calculated in accordance with the following formula, using the found weights W2 and W3.

$$\text{Absorbency against pressure 0.3 psi (AAP 0.3)}$$
$$= (W3 \text{ (g)} - W2 \text{ (g)})/(\text{Weight of water absorbing resin or water absorbing agent (0.900 g)})$$

(3) Weight average particle diameter (D50) and logarithmic standard deviation of particle diameter distribution ($\sigma\zeta$)

A given water absorbing resin was classified with JIS standard sieves (JIS Z 8801-1 (2550)) measuring 850 $\mu$m, 710 $\mu$m, 600 $\mu$m, 500 $\mu$m, 425 $\mu$m, 300 $\mu$m, 212 $\mu$m, 150 $\mu$m, 106 $\mu$m, and 45 $\mu$m in aperture and the residual percentages R consequently found were plotted on a logarithmic probability paper. The particle diameter corresponding to R = 50 weight% was read out as the weight average particle diameter (D50). Let X1 stand for the particle diameter for R = 84.1% and X2 the particle diameter for R = 15.9% respectively, and the logarithmic standard deviation ($\sigma\zeta$) will be expressed by the following formula. The magnitude ($\sigma\zeta$) signifies that the particle size distribution narrows in accordance as this magnitude decreases.

$$\sigma\varsigma = 0.5 \times \ln (X2/X1)$$

The classification adopted in determining the logarithmic standard deviation ($\sigma\zeta$) in the particle size distribution was effected by charging the JIS standard sieves (the Iida Testing Sieves: diameter 8 cm) measuring 850 $\mu$m, 710 $\mu$m, 600 $\mu$m, 500 $\mu$m, 425 $\mu$m, 300 $\mu$m, 212 $\mu$m, 150 $\mu$m, 106 $\mu$m, and 45 $\mu$m in aperture with 10.0 g of a given water absorbing resin under the conditions of room temperature (20 to 25°C) and humidity 50RH% and shaking the sieves with a shaking classifier (Iida Sieve Shaker, Type ES-65, Ser No. 501).

(4) Eluted soluble content

In a plastic container having an inner volume of 250 ml and furnished with a lid, 184.3 g of an aqueous 0.9 weight% saline solution was placed, 1.00 g of a given water absorbing resin or water absorbing agent was added to the aqueous solution, and they were stirred by rotating a stirrer so as to extract an eluted soluble component from the resin for 16 hours. The extracted liquid was filtered with one filter paper (made by ADVANTEC Toyo K.K. and sold under the product name "JIS P 3801, No. 2," and having a thickness of 0.26 mm and a retention particle diameter of 5 m). The portion 50.0 g of the resultant filtrate was taken as a test solution.

First, 50.0 g of an aqueous 0.9 weight% saline solution alone was titrated first with an aqueous 0.1N NaOH solution till pH 10 and then with an aqueous 0.1N HCl solution till pH 2.7 to obtain titers ([bNaOH] ml and [bHCl] ml).

By performing the same titrating operation on the test solution, the titers ([NaOH] ml and [HCl] ml) were obtained.

In the case of a water absorbing resin formed of known amounts of acrylic acid and a sodium salt thereof, for example, the eluted soluble content of the water absorbing resin was calculated in accordance with the following formula, based on the average molecular weight of the monomer and the titers found by the aforementioned operation. When the amounts mentioned above were unknown, the average molecular weight of the monomer was calculated by using the neutralization degree determined by titration.

$$\text{Eluted soluble content (weight\%)} = 0.1 \times (\text{average molecular weight of monomer})$$
$$\times 184.3 \times 100 \times ([\text{HCl}] - [\text{bHCl}])/1000/1.0/50.0$$

$$\text{Neutralization degree (mol\%)} = (1 - ([\text{NaOH}] - [\text{bNaOH}])/([\text{HCl}] - [\text{bHCl}])) \times 100$$

(5) Water content

In an aluminum cup measuring 4 cm in diameter of bottom surface and 2 cm in height, 1.00 g of a given water absorbing resin was uniformly scattered on the bottom surface of the aluminum cup. The aluminum cup containing the absorbing agent was weighed to find the weight W4 (g). It was left standing in a hot air drier adjusted at 180°C for 3 hours. The agent-containing aluminum cup was weighed to find the weight W5 (g) immediately after it was taken out of the hot air drier (within less than 1 minute). The water content (weight%) was calculated in accordance with the following formula using these weights W4 and W5.

$$\text{Water content (weight\%)} = (W4\,(g) - W5\,(g))/(\text{weight (g) of water absorbing resin}) \times 100$$

(6) Surface tension of mixing aid solution

In a thoroughly washed 100 mL beaker, 40 mL of purified water of 20°C was placed and the purified water was tested for surface tension by the use of a surface tension meter (made by Kruss Corp and sold under the trademark designation of "K11 Automatic Surface Tension Meter"). In this determination, the magnitude of surface tension must be in the range of 0.00072 - 0.00074 N/cm (72 to 74 dynes/cm). Then, the mixing aid solution adjusted to 20°C was tested for surface tension by following the same procedure. Incidentally, this invention adopted the plate method using a platinum plate, which was thoroughly cleaned with water prior to each determination and thermally cleaned with a burner prior to use.

(Referential Example 1)

In a reaction vessel formed by attaching a stopper to a jacketed stainless steel twin arm type kneader furnished with two sigma type vanes and having an inner volume of 10 liters, 8.55 g of polyethylene glycol diacrylate was dissolved in 5446 g of an aqueous sodium acrylate solution having the monomer concentration of 39 weight% and the neutralization degree of 60 mol% to prepare a reaction solution. Then, this reaction solution was deaerated in an atmosphere of nitrogen gas for 30 minutes. When 20.1 g of an aqueous 10 weight% sodium persulfate solution and 25.2 g of an aqueous 0.1 weight% L-ascorbic acid solution were subsequently added as kept stirred to the reaction solution, polymerization was initiated within about one minute of adding the aqueous solutions. Then, the polymerization was carried out at 20 to 95°C while the formed gel was continuously pulverized. After the elapse of 30 minutes subsequent to the start of polymerization, a hydrogel-like cross-linked polymer was taken out. The produced hydrogel-like cross-linked polymer was in a form finely divided into particulates not more than about 5 mm in diameter. This finely divided hydrogel-like cross-linked polymer was spread on a 50-mesh metal gauze (aperture 300 μm) and dried with hot air at 175°C for 50 minutes. Thus, a water absorbing resin (A) formed of indeterminately shaped, easily pulverizable aggregates of a particulate or powdery dry product was obtained.

The water absorbing resin (A) was pulverized with a roll mill and further classified with a JIS standard sieve having an aperture of 710 μm. Then, the particles which had passed the apertures of 710 μm in the foregoing operation were classified with a JIS standard sieve having an aperture of 150 μm to remove the water absorbing resin particles which had passed the JIS standard sieve of an aperture of 150 μm. Thus, a water absorbing resin (A1) was obtained. The water absorbing resin (A1) was rated to determine various properties. As a result of the rating, physical properties shown in Table 1 and the various particle size distributions shown in Table 2 were obtained.

(Referential Example 2)

In a reaction vessel formed by attaching a stopper to a jacketed stainless steel twin arm type kneader furnished with two sigma type vanes and having an inner volume of 10 liters, 7.95 g of polyethylene glycol diacrylate was dissolved in 5438 g of an aqueous sodium acrylate solution having the monomer concentration of 39 weight% and the neutralization degree of 70 mol% to prepare a reaction solution. Then, this reaction solution was deaerated in an atmosphere of nitrogen gas for 30 minutes. When 29.43 g of an aqueous 10 weight% sodium persulfate solution and 24.53 g of an aqueous 0.1 weight% L-ascorbic acid solution were subsequently added as kept stirred to the reaction solution, polymerization was initiated within about one minute of adding the aqueous solutions. Then, the polymerization was carried out at 20 to 95°C while the formed gel was continuously pulverized. After the elapse of 30 minutes subsequent to the start of polymerization, a hydrogel-like cross-linked polymer was taken out. The produced hydrogel-like cross-linked polymer was in a form finely divided into particulates not more than about 5 mm in diameter. This finely divided hydrogel-like cross-linked polymer was spread on a 50-mesh metal gauze (aperture 300 m) and dried with hot air at 175°C for 50 minutes. Thus, a water absorbing resin (B) formed of indeterminately shaped, easily pulverizable aggregates of a particulate or powdery dry product was obtained.
The water absorbing resin (B) was pulverized with a roll mill and further classified with a JIS standard sieve having an aperture of 710 $\mu$m. Then, the particles which had passed the apertures of 710 $\mu$m in the foregoing operation were classified with a JIS standard sieve having an aperture of 150 $\mu$m to remove the water absorbing resin particles which had passed the JIS standard sieve of an aperture of 150 $\mu$m. Thus, a water absorbing resin (B1) was obtained. The water absorbing resin (B1) was rated to determine various properties. As a result of the rating, physical properties shown in Table 1 and the various particle size distributions shown in Table 2 were obtained.

(Referential Example 3)

In a reaction vessel formed by attaching a stopper to a jacketed stainless steel twin arm type kneader furnished with two sigma type vanes and having an inner volume of 10 liters, 9.39 g of polyethylene glycol diacrylate was dissolved in 5447 g of an aqueous sodium acrylate solution having the monomer concentration of 39 weight% and the neutralization degree of 80 mol%. to prepare a reaction solution. Then, this reaction solution was deaerated in an atmosphere of nitrogen gas for 30 minutes. When 19.1 g of an aqueous 10 weight% sodium persulfate solution and 23.9 g of an aqueous 0.1 wight% L-ascorbic acid solution were subsequently added as kept stirred to the reaction solution, polymerization was initiated within about one minute of adding the aqueous solutions. Then, the polymerization was carried out at 20 to 95°C while the formed gel was continuously pulverized. After the elapse of 30 minutes subsequent to the start of polymerization, a hydrogel-like cross-linked polymer was taken out. The produced hydrogel-like cross-linked polymer was in a form finely divided into particulates not more than about 5 mm in diameter. This finely divided hydrogel-like cross-linked polymer was spread on a 50-mesh metal gauze (aperture 300 m) and dried with hot air at 175°C for 50 minutes. Thus, a water absorbing resin (C) formed of indeterminately shaped, easily pulverizable aggregates of a particulate or powdery dry product was obtained.
The water absorbing resin (C) was pulverized with a roll mill and further classified with a JIS standard sieve having an aperture of 710 $\mu$m. Then, the particles which had passed the apertures of 710 $\mu$m in the foregoing operation were classified with a JIS standard sieve having an aperture of 150 $\mu$m to remove the water absorbing resin particles which had passed the JIS standard sieve of an aperture of 150 $\mu$m. Thus, a water absorbing resin (C1) was obtained. The water absorbing resin (C1) was rated to determine various properties. As a result of the rating, physical properties shown in Table 1 and the various particle size distributions shown in Table 2 were obtained.

(Referential Example 4)

In a reaction vessel formed by attaching a stopper to a jacketed stainless steel twin arm type kneader furnished with two sigma type vanes and having an inner volume of 10 liters, 6.04 g of polyethylene glycol diacrylate was dissolved in 5452 g of an aqueous sodium acrylate solution having the monomer concentration of 39 weight% and the neutralization degree of 90 mol to prepare a reaction solution. Then, this reaction solution was deaerated in an atmosphere of nitrogen gas for 30 minutes. When 19.1 g of an aqueous 10 weight% sodium persulfate solution and 23.9 g of an aqueous 0.1 weight% L-ascorbic acid solution were subsequently added as kept stirred to the reaction solution, polymerization was initiated within about one minute of adding the aqueous solutions. The polymerization was carried out at 20 to 95°C while the formed gel was continuously pulverized. After the elapse of 30 minutes subsequent to the start of polymerization, a hydrogel-like cross-linked polymer was taken out. The produced hydrogel-like cross-linked polymer was in a form finely divided into particulates not more than about 5 mm in diameter. This finely divided hydrogel-like cross-linked polymer was spread on a 50-mesh metal gauze (aperture 300 $\mu$m) and dried with hot air at 175°C for 50 minutes. Thus, a water absorbing resin (D) formed of indeterminately shaped, easily pulverizable aggregates of a particulate or powdery dry product was obtained.

The water absorbing resin (D) was pulverized with a roll mill and further classified with a JIS standard sieve having an aperture of 710 μm. Then, the particles which had passed the apertures of 710 μm in the foregoing operation were classified with a JIS standard sieve having an aperture of 150 μm to remove the water absorbing resin particles which had passed the JIS standard sieve of an aperture of 150 μm. Thus, a water absorbing resin (D1) was obtained. The water absorbing resin (D1) was rated to determine various properties. As a result of the rating, physical properties shown in Table 1 and the various particle size distributions shown in Table 2 were obtained.

(Referential Example 5)

The water absorbing resin (A) described in Referential Example 1 was pulverized with a pin mill and further classified with the JIS sieve having an aperture of 500 μm to remove the water absorbing resin particles which do not pass on the JIS sieve having an aperture of 500 μm. Thus, a water absorbing resin (A2) was obtained. This water absorbing resin (A2) was rated to determine various properties. As a result of the rating, physical properties shown in Table 1 and the various particle size distributions shown in Table 2 were obtained.

(Referential Example 6)

The water absorbing resin (A) described in Referential Example 1 was pulverized with a pin mill and further classified with the JIS sieve having an aperture of 710 μm to remove the water absorbing resin particles which do not pass on the J1S sieve having an aperture of 710 μm . Thus, a water absorbing resin (A3) was obtained. This water absorbing resin (A3) was rated to determine various properties. As a result of the rating, physical properties shown in Table 1 and the various particle size distributions shown in Table 2 were obtained.

(Example 1)

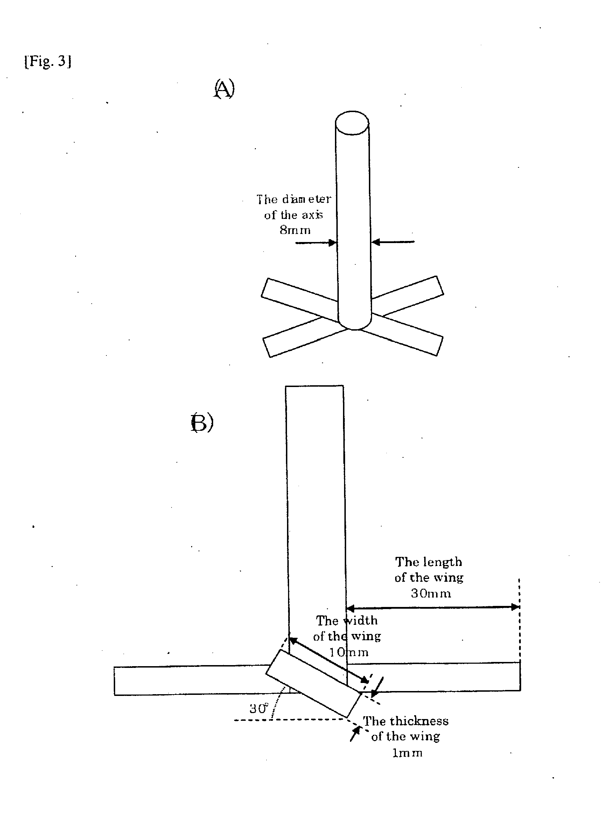

In a separable flask made of quartz (illustrated in Fig. 2), 10 g of the water absorbing resin (A1) was placed and stirred with stirring vanes (illustrated in Fig. 3) at 500 rpm meanwhile 0.85 g of an aqueous 5.88 weight% polyethylene glycol methyl ether Mn 2000 (PEG-OMe 2000: $CH_3(OCH_2CH_2)_nOH$, number average molecular weight Mn: 20000, made by Aldrich Corp.) solution was added thereto. After the stirring was continued for 10 minutes, the stirred mixture was irradiated with an ultraviolet rays at a radiation intensity of 60 mW/cm$^2$ for 10 minutes at room temperature by using an ultraviolet rays radiating device (made by Ushio Denki K.K. and sold under the product code of "UV-152/IMNSC3-AA06") furnished with a metal halide lamp (made by the same company and sold under the product code of "UVL-1500M2-N1") to obtain a surface-treated water absorbing agent (1).

The conditions for the synthesis of the produced water absorbing agent (1) are shown in Table 3 and the results of the test of this agent for various physical properties are shown in Table 4.

The various physical properties concerning "the water contents of 8 weight% corrected" indicated in Table 4 were the magnitudes calculated in accordance with the following formula.

$$CRC \text{ (g/g) after correction of water content of 8 weight\%} = (CRC \text{ (g/g) with no correction of water content} + 1)/0.92 - 1$$

$$AAP \text{ } 0.3 \text{ (g/g) after correction of water content of 8 weight\%} = AAP \text{ } 0.3 \text{ (g/g) with no correction of water content}/0.92$$

$$\text{Eluted soluble content (weight\%) after correction o water content of 8 weight\%} = \text{Eluted soluble content with no correction of water content (weight\%)}/0.92$$

(Example 2)

A surface-treated water absorbing agent (2) was obtained by following the procedure of Example 1 while using 10 g of the water absorbing resin (B1) instead and changing the rotational frequency of the stirring vanes to 450 rpm. The conditions for the synthesis of the produced water absorbing agent (2) are shown in Table 3 and the results of the test of this agent for various physical properties are shown in Table 4.

(Example 3)

A surface-treated water absorbing agent (3) was obtained by following the procedure of Example 1 while using 10 g of the water absorbing resin (C1) instead. The conditions for the synthesis of the produced water absorbing agent (3) are shown in Table 3 and the results of the test of this agent for various physical properties are shown in Table 4.

(Example 4)

A surface-treated water absorbing agent (4) was obtained by following the procedure of Example 1 while using 10 g of the water absorbing resin (D1) instead. The conditions for the synthesis of the produced water absorbing agent (4) are shown in Table 3 and the results of the test of this agent for various physical properties are shown in Table 4.

(Example 5)

A surface-treated water absorbing agent (5) was obtained by following the procedure of Example 1 while using 0.85 g of an aqueous 5.88 weight% polyethylene glycol methyl ether Mn 550 (PEG-OMe 550: $CH_3(OCH_2CH_2)_nOH$, number average molecular weight Mn: 550, made by Aldrich Corp.) solution instead and changing the rotational frequency of the stirring vanes to 400 rpm. The conditions for the synthesis of the produced water absorbing agent (5) are shown in Table 3 and the results of the test of this agent for various physical properties are shown in Table 4.

(Example 6)

A surface-treated water absorbing agent (6) was obtained by following the procedure of Example 1 while using 0.85 g of an aqueous 5.88 weight% polyethylene glycol methyl ether Mn 5000 (PEG-OMe 5000: $CH_3(OCH_2CH_2)_nOH$, number average molecular weight Mn: 5000, made by Aldrich Corp.) solution instead and changing the rotational frequency of the stirring vanes to 400 rpm. The conditions for the synthesis of the produced water absorbing agent (6) are shown in Table 3 and the results of the test of this agent for various physical properties are shown in Table 4.

(Example 7)

A surface-treated water absorbing agent (7) was obtained by following the procedure of Example 1 while using 0.85 g of an aqueous 5.88 weight% polyethylene glycol Mn 600 (PEG 600:$H(OCH_2CH_2)_nOH$, number average molecular weight Mn: 600, made by Wako Junyaku Kogyo K.K.) solution instead and changing the rotational frequency of the stirring vanes to 600 rpm. The conditions for the synthesis of the produced water absorbing agent (7) are shown in Table 3 and the results of the test of this agent for various physical properties are shown in Table 4.

(Example 8)

A surface-treated water absorbing agent (8) was obtained by following the procedure of Example 1 while using 0.85 g of an aqueous 5.88 weight% polyethylene glycol Mn 2000 (PEG 200: $H(OCH_2CH_2)_nOH$, number average molecular weight Mn: 2000, made by Wako Junyaku Kogyo K.K.) solution instead and changing the rotational frequency of the stirring vanes to 600 rpm. The conditions for the synthesis of the produced water absorbing agent (8) are shown in Table 3 and the results of the test of this agent for various are shown in Table 4.

(Example 9)

A surface-treated water absorbing agent (9) was obtained by following the procedure of Example 1 while using 0.85 g of an aqueous 5.88 weight% polyethylene glycol Mn 6000 (PEG 6000: $H(OCH_2CH_2)_nOH$, number average molecular weight Mn: 6000, made by Kishida Kagaku K.K.) solution instead and changing the rotational frequency of the stirring vanes to 600 rpm. The conditions for the synthesis of the produced water absorbing agent (9) are shown in Table 3 and the results of the test of this agent for various physical properties are shown in Table 4.

(Comparative Example 1)

In a separable flask made of quartz, 10 g of the water absorbing resin (A1) was placed, stirred with stirring vanes at 500 rpm, and irradiated with an ultraviolet rays at a radiation intensity of 60 mW/cm² for 10 minutes at room temperature by using an ultraviolet rays radiating device (made by Ushio Denki K.K. and sold under the product code of "UV-152/IMNSC3-AA06") furnished with a metal halide lamp (made by the same company and sold under the product code of "UVL-1500M2-N1") to obtain a comparative surface-treated water absorbing agent (1). The conditions for the synthesis of the produced comparative water absorbing agent (1) are shown in Table 3 and the results of the test of this agent for various physical properties are shown in Table 4.

(Comparative Example 2)

A comparative water absorbing agent (2) was obtained by following the procedure of Comparative Example 1 while using 10 g of the water absorbing resin (B1) instead. The conditions for the synthesis of the produced comparative water absorbing agent (2) are shown in Table 3 and the results of the test of this agent for various physical properties are shown in Table 4.

(Comparative Example 3)

In a separable flask made of quartz, 10 g of the water absorbing resin (B1) was placed and stirred with stirring vanes at 450 rpm meanwhile 1.30 g of an aqueous 38.5 weight% ammonium persulfate (APS) solution was added thereto. After the stirring was continued for 10 minutes, the stirred mixture was irradiated with an ultraviolet rays at a radiation intensity of 60 mW/cm$^2$ for 10 minutes at room temperature by using an ultraviolet rays radiating device (made by Ushio Denki K.K. and sold under the product code of "UV-152/IMNSC3-AA06") furnished with a metal halide lamp (made by the same company and sold under the product code of "UVL-1500M2-N1") to obtain a comparative surface-treated water absorbing agent (3). The conditions for the synthesis of the produced comparative water absorbing agent (3) are shown in Table 3 and the results of the test of this agent for various physical properties are shown in Table 4.

(Comparative Example 4)

In a separable flask made of quartz, 10 g of the water absorbing resin (A1) was placed and stirred with stirring vanes at 450 rpm meanwhile 0.80 g of purified water was added thereto. A comparative water absorbing agent (4) was obtained by continuing the stirring for 10 minutes. The conditions for the synthesis of the produced comparative water absorbing agent (4) are shown in Table 3 and the results of the test of this agent for various physical properties are shown in Table 4.

(Comparative Example 5)

In a separable flask made of quartz, 10 g of the water absorbing resin (A1) was placed and stirred with stirring vanes at 450 rpm meanwhile 0.85 g of an aqueous 5.88 weight% polyethylene glycol methyl ether Mn 2000 ($CH_3$($OCH_2CH_2$)$_n$OH, number average molecular weight Mn: 2000, made by Aldrich Corp.) solution. A comparative water absorbing agent (5) was obtained by continuing the stirring for 10 minutes. The conditions for the synthesis of the produced comparative water absorbing agent (5) are shown in Table 3 and the results of the test of the agent for various physical properties are shown in Table 4.

(Comparative Example 6)

A comparative water absorbing agent (6) having surface treated part of the water absorbing resin thereof was obtained by following the procedure of Example 1 while using 0.80g of a purified water in the place of the aqueous 5.88 weight% polyethylene glycol methyl ether and omitting the rotation with the stirring vanes during the radiation of the ultraviolet rays. The conditions for the synthesis of the produced comparative water absorbing agent (6) are shown in Table 3 and the results of the test of the agent for various physical properties are shown in Table 4.

(Comparative Example 7)

A comparative water absorbing agent (7) having surface treated part of the water absorbing resin thereof was obtained by following the procedure of Example 1 while omitting the rotation with the stirring vanes during the radiation of the ultraviolet rays. The conditions for the synthesis of the produced comparative water absorbing agent (7) are shown in Table 3 and the results of the test of the agent for various physical properties are shown in Table 4.

(Comparative Example 8)

In a separable flask made of quartz, 10 g of the water absorbing resin (A2) was placed and stirred with stirring vanes at 450 rpm meanwhile 0.80 g of purified water was added thereto. After the stirring was continued for 10 minutes, the stirred mixture was irradiated with an ultraviolet rays at a radiation intensity of 60 mW/cm$^2$ for 10 minutes at room temperature by using an ultraviolet rays radiating device (made by Ushio Denki K.K. and sold under the product code of "UV-152/IMNSC3-AA06") furnished with a metal halide lamp (made by the same company and sold under the product code of "UVL-1500M2-N1") to obtain a comparative surface-treated water absorbing agent (8). The conditions for the synthesis of the produced comparative water absorbing agent (8) are shown in Table 3 and the results of the test of this agent for various physical properties are shown in Table 4.

(Comparative Example 9)

A comparative water absorbing agent (9) was obtained by following the procedure of Comparative Example 8 while using 10 g of the water absorbing resin (A3) instead. The conditions for the synthesis of the produced comparative water absorbing agent (9) are shown in Table 3 and the results of the test of this agent for various physical properties are shown in Table 4.

[Table 1]

| Water absorbing resin | Neutralization degree (mol%) | CRC (g/g) | AAP 0.3 (g/g) | Water content (weight%) | Eluted soluble content (weight%) |
|---|---|---|---|---|---|
| Water absorbing resin (A1) | 60 | 35.1 | 12.6 | 5.7 | 13.3 |
| Water absorbing resin (B1) | 70 | 34.5 | 11.8 | 6.3 | 12.2 |
| Water absorbing resin (C1) | 80 | 32.4 | 14.2 | 5.5 | 6.7 |
| Water absorbing resin (D1) | 90 | 34.6 | 6.6 | 6.1 | 11.4 |
| Water absorbing resin (A2) | 60 | 33.9 | 10.1 | | - |
| Water absorbing resin (A3) | 60 | 34.1 | 10.3 | | - |

[Table 2]

| Referential Example | 1 | 2 | 3 | 4 | 5 | 6 |
|---|---|---|---|---|---|---|
| Water absorbing resin | A1 | B1 | C1 | D1 | A2 | A3 |
| D50 (m) | 345 | 346 | 322 | 323 | 231 | 242 |
| σζ | 0.327 | 0.332 | 0.359 | 0.339 | 0.413 | 0.472 |
| Particle size distribution | | | | | | |
| Not less than 850 μm (weight%) | 0.0 | 0.0 | 0.0 | 0.0 | 0.0 | 0.0 |
| 850 to 710 μm (weight%) | 0.1 | 0.2 | 0.0 | 0.0 | 0.0 | 0.0 |
| 710 to 600 μm (weight%) | 1.0 | 1.5 | 0.0 | 0.0 | 0.0 | 0.8 |
| 600 to 500 μm (weight%) | 3.7 | 4.1 | 2.7 | 1.9 | 0.0 | 2.3 |
| 500 to 425 μm (weight%) | 21.7 | 22.6 | 19.2 | 18.8 | 7.1 | 8.8 |
| 425 to 300 μm (weight%) | 39.6 | 37.1 | 36.2 | 37.8 | 18.9 | 19.4 |
| 300 to 212 μm (weight%) | 23.0 | 23.9 | 26.6 | 28.1 | 32.4 | 30.7 |

(continued)

| Referential Example | 1 | 2 | 3 | 4 | 5 | 6 |
|---|---|---|---|---|---|---|
| 212 to 150 μm (weight%) | 9.3 | 9.3 | 12.6 | 10.3 | 26.7 | 22.5 |
| 150 to 45 μm (weight%) | 1.5 | 1.3 | 2.6 | 3.1 | 12.6 | 13.1 |
| Not more than 45 μm (weight%) | 0.1 | 0.0 | 0.1 | 0.0 | 2.3 | 2.4 |
| Total (weight%) | 100.0 | 100.0 | 100.0 | 100.0 | 100.0 | 100.0 |

(Not less than A μm) This indicates a water absorbing resin which does not pass a sieve of an aperture of A μm during classification.
(Not more than B μm) This indicates a water absorbing resin passed through a sieve of an aperture of B μm during classification.
(C - D μm) This indicates a water absorbing resin passed through a sieve of an aperture of C μm and does not pass a sieve of an aperture of D μm during classification.

[Table 3]

| Example No. | Water absorbing agent | Additive composition and ratio thereof (weight%) to water absorbing resin (A1 - D1) | Surface tension of additive solution (dynes/cm) | Rotational frequency of stirring vanes during radiation of UV (rpm) | Duration of UV radiation |
|---|---|---|---|---|---|
| Example 1 | Water absorbing agent (1) | W*)/PEG-OMe2000=8/0.5 | 51.7 | 500 | 10 minutes |
| Example 2 | Water absorbing agent (2) | W*)/PEG-OMe2000=8/0.5 | 51.7 | 450 | 10 minutes |
| Example 3 | Water absorbing agent (3) | W*)/PEG-OMe2000=8/0.5 | 51.7 | 500 | 10 minutes |
| Example 4 | Water absorbing agent (4) | W*)/PEG-OMe2000=8/0.5 | 51.7 | 500 | 10 minutes |
| Example 5 | Water absorbing agent (5) | W*)/PEG-OMe550=8/0.5 | | 400 | 10 minutes |
| Example 6 | Water absorbing agent (6) | W*)/PEG-OMe5000=8/0.5 | | 400 | 10 minutes |
| Example 7 | Water absorbing agent (7) | W*) /PEG600=8/0.5 | | 600 | 10 minutes |
| Example 8 | Water absorbing agent (8) | W*) /PEG2000=8/0.5 | | 600 | 10 minutes |
| | | | | | |
| Example 9 | Water absorbing agent (9) | W*) /PEG6000=8/0.5 | | 600 | 10 minutes |

(continued)

| Example No. | Water absorbing agent | Additive composition and ratio thereof (weight%) to water absorbing resin (A1 - D1) | Surface tension of additive solution (dynes/cm) | Rotational frequency of stirring vanes during radiation of UV (rpm) | Duration of UV radiation |
|---|---|---|---|---|---|
| Comparative Example 1 | Comparative Water absorbing agent (1) | - | | 500 | 10 minutes |
| Comparative Example 2 | Comparative Water absorbing agent (2) | - | | 500 | 10 minutes |
| Comparative Example 3 | Comparative Water absorbing agent (3) | W*)/APS=8/5 | | 450 | 10 minutes |
| Comparative Example 4 | Comparative Water absorbing agent (4) | W*)=8 | 73 | | |
| Comparative Example 5 | Comparative Water absorbing agent (5) | W*)/PEG-OMe2000=8/0.5 | 51.7 | | |
| Comparative Example 6 | Comparative Water absorbing agent (6) | W*)=8 | 73 | 0 | 10 minutes |
| Comparative Example 7 | Comparative Water absorbing agent (7) | W*)/PEG-OMe2000=8/0.5 | 51.7 | 0 | 10 minutes |
| Comparative Example 8 | Comparative Water absorbing agent (8) | W*)=8 | 73 | 500 | 10 minutes |
| Comparative Example 9 | Comparative Water absorbing agent (9) | W*)=8 | 73 | 500 | 10 minutes |
| *)W: purified water | | | | | |

[Table 4]

| Example No. | Water absorbing agent | Water content not corrected | | | Water content of 8 weight% corrected** | | |
|---|---|---|---|---|---|---|---|
| | | CRC (g/g) | AAP0.3 (g/g) | Eluted soluble content (weight%) | CRC (g/g) | AAP0.3 (g/g) | Eluted soluble content (weight %) |
| Example 1 | Water absorbing agent (1) | 32.5 | 27.8 | 12.8 | 35.4 | 30.2 | 14.0 |
| Example 2 | Water absorbing agent (2) | 31.6 | 26.5 | 11.8 | 34.4 | 28.8 | 12.8 |
| Example 3 | Water absorbing agent (3) | 28.8 | 25.4 | - | 31.4 | 27.6 | - |
| Example 4 | Water absorbing agent (4) | 32.3 | 18.6 | - | 35.2 | 20.2 | - |
| Example 5 | Water absorbing agent (5) | 32.5 | 28.8 | - | 35.7 | 31.3 | - |
| Example 6 | Water absorbing agent (6) | 31.9 | 27.1 | - | 34.8 | 29.5 | - |
| Example 7 | Water absorbing agent (7) | 32.9 | 28.2 | - | 35.8 | 30.7 | - |
| Example 8 | Water absorbing agent (8) | 33 | 28.0 | - | 36.0 | 30.4 | - |
| Example 9 | Water absorbing agent (9) | 32.1 | 27.0 | - | 35.0 | 29.3 | - |
| Comparative Example 1 | Comparative Water absorbing agent (1) | 37.6 | 9.6 | 15.6 | - | - | - |
| Comparative Example 2 | Comparative Water absorbing agent (2) | 36.1 | 8.2 | 13.5 | - | - | - |
| Comparative Example 3 | Comparative Water absorbing agent (3) | 24.0 | 26.1 | | 26.2 | 28.4 | |
| Comparative Example 4 | Comparative Water absorbing agent (4) | 32.7 | 11.9 | | 35.6 | 12.9 | - |

(continued)

| Example No. | Water absorbing agent | Water content not corrected | | | Water content of 8 weight% corrected** | | |
|---|---|---|---|---|---|---|---|
| | | CRC (g/g) | AAP0.3 (g/g) | Eluted soluble content (weight%) | CRC (g/g) | AAP0.3 (g/g) | Eluted soluble content (weight %) |
| Comparative Example 5 | Comparative Water absorbing agent (5) | 33.4 | 11.5 | | 36.4 | 12.5 | - |
| Comparative Example 6 | Comparative Water absorbing agent (6) | 32.7 | 14.2 | | 35.6 | 15.4 | - |
| Comparative Example 7 | Comparative Water absorbing agent (7) | 32.2 | 14.8 | | 35.1 | 16.1 | - |
| Comparative Example 8 | Comparative Water absorbing agent (8) | 31.6 | 14.1 | - | 34.3 | 15.3 | - |
| Comparative Example 9 | Comparative Water absorbing agent (9) | 31.2 | 14.8 | - | 33.9 | 15.7 | - |
| **) Water content corrected: Water content of water absorbing resin as determined, and water content of **water absorbing agent** is corrected by the weight of added water (8 weight%). | | | | | | | |

The above Examples are to more specifically explain the present invention, and the present invention should not be construed to be limited to the Examples.

This invention, in modifying a water absorbing resin, is capable of giving to the resin a satisfactory surface treatment even at a reaction temperature in the neighborhood of room temperature. The water absorbing agent consequently obtained excels in water absorption properties and, therefore, is industrially useful as evinced by being utilized as for disposable diapers.

The present application is based on Japanese Patent Application No. 2005-270761 filed on September 16, 2005.

**Claims**

1. A method of making an absorbent member for use in absorbent articles, said absorbent member comprising an absorbing agent made by a method comprising the steps of

   a) mixing a water absorbing resin, water, and a mixing aid without adding a water-soluble radical polymerization initiator and an ethylenically unsaturated monomer and
   b) irradiating the resultant mixture with ultraviolet rays while still agitating the mixture in order to maintain a mixed state,

   wherein the irradiation time is not less than 0.1 minute and less than 60 minutes, wherein in said method the aqueous solution has a surface tension of from 0.0004 N/cm to 0.00075 N/cm (40 dynes/cm to 75 dynes/cm), and wherein the absorbency of physiological saline against pressure of 2.07 kPa of the water absorbing agent is at least 15 g/g and is less than 50 g/g and is increased by at least 1 g/g comparing with that of the water absorbing resin.

**2.** The method according to claim 1, wherein in said method the amount of the water to be mixed is at least 1 weight part and is less than 50 weight parts based on 100 weight parts of the water absorbing resin.

**3.** The method according to claim 1 or claim 2, wherein in said method the mixing aid is at least one compound selected from the group consisting of surfactants, water-soluble polymer, hydrophilic organic solvents, water-soluble inorganic compounds, inorganic acid salts, organic acid salts.

**4.** The method according to any of claims 1 to 3, wherein in said method the mixing aid is added in an amount of at least 0.01 weight parts and less than 50 weight parts, based on 100 parts by weight of the water absorbent resin.

**5.** The method according to any of claims 1 to 4, wherein in said method the mixing aid is mixed in the form of an aqueous solution with the water absorbing resin.

**6.** The method according to any of claims 3 to 5, wherein in said method the surfactant is polyoxyethylene alkyl ether.

**7.** The method according to any of claims 3 to 5, wherein in said method the surfactant is polyethylene glycol.

**8.** The method according to any of claims 3 to 5, wherein in said method the water-soluble inorganic compound is a water-soluble metal salt.

**9.** The method according to any of claims 1 to 8, wherein the water absorbing resin contains particles of a particle diameter of at least 150 $\mu$m and less than 850 $\mu$m in a ratio of 90 weight% to 100 weight% of all the particles therein and has a weight average particle diameter (D50) of at least 300 $\mu$m and less than 500 $\mu$m and a logarithmic standard deviation ($\sigma\zeta$) of particle distribution of from 0.20 to 0.45.

**10.** The method according to any of claims 1 to 9, wherein the eluted soluble content of the water absorbing agent is at least 0.1 weight parts and is less than 30 weight parts, based on 100 weight parts of the water absorbing resin.

**11.** The method according to any of claims 1 to 10, wherein the water absorbing resin is obtained by polymerizing a monomer having acrylic acid (salt) as a main component thereof.


**Patentansprüche**

**1.** Verfahren zum Herstellen eines absorbierenden Elements zum Gebrauch in Absorptionsartikeln, wobei das absorbierende Element ein Absorptionsmittel umfasst, das durch ein die folgenden Schritte umfassendes Verfahren hergestellt wird:

 a) Mischen eines wasserabsorbierenden Harzes, Wasser und eines Mischhilfsmittels ohne Hinzufügen eines wasserlöslichen radikalischen Polymerisationsinitiators und eines ethylenisch ungesättigten Monomers und
 b) Bestrahlen der resultierenden Mischung mit ultravioletten Strahlen unter weiterem Rühren der Mischung, um weiterhin einen gemischten Zustand beizubehalten,

wobei die Bestrahlungszeit mindestens 0,1 Minuten und höchstens 60 Minuten beträgt, wobei in dem Verfahren die wässrige Lösung eine Oberflächenspannung von 0,0004 N/cm bis 0,00075 N/cm (40 Dyn/cm bis 75 Dyn/cm) aufweist und wobei die Absorption von physiologischer Kochsalzlösung durch das wasserabsorbierende Mittel bei einem Druck von 2,07 kPa mindestens 15 g/g und höchstens 50 g/g beträgt und um mindestens 1 g/g höher ist als bei dem wasserabsorbierenden Harz.

**2.** Verfahren nach Anspruch 1, wobei in dem Verfahren die beigemischte Menge an Wasser mindestens 1 Gewichtsteil und höchstens 50 Gewichtsteile ausgehend von 100 Gewichtsteilen an wasserabsorbierendem Harz beträgt.

**3.** Verfahren nach Anspruch 1 oder Anspruch 2, wobei in dem Verfahren das Mischhilfsmittel mindestens eine Verbindung ausgewählt aus der Gruppe bestehend aus Tensiden, wasserlöslichem Polymer, hydrophilen organischen Lösemitteln, wasserlöslichen anorganischen Verbindungen, anorganischen Säuresalzen, organischen Säuresalzen ist.

**4.** Verfahren nach einem der Ansprüche 1 bis 3, wobei in dem Verfahren das Mischhilfsmittel in einer Menge von mindestens 0,01 Gewichtsteilen und weniger als 50 Gewichtsteilen ausgehend von 100 Gewichtsteilen an wasserabsorbierendem Harz beträgt.

5. Verfahren nach einem der Ansprüche 1 bis 4, wobei in dem Verfahren das Mischhilfsmittel in Form einer wässrigen Lösung mit dem wasserabsorbierenden Harz vermischt wird.

6. Verfahren nach einem der Ansprüche 3 bis 5, wobei das Tensid in dem Verfahren Polyoxyethylenalkylether ist.

7. Verfahren nach einem der Ansprüche 3 bis 5, wobei das Tensid in dem Verfahren Polyethylenglykol ist.

8. Verfahren nach einem der Ansprüche 3 bis 5, wobei die wasserlösliche anorganische Verbindung in dem Verfahren ein wasserlösliches Metallsalz ist.

9. Verfahren nach einem der Ansprüche 1 bis 8, wobei das wasserabsorbierende Harz Partikel mit einem Partikeldurchmesser von mindestens 150 $\mu$m und weniger als 850 $\mu$m in einem Verhältnis von 90 Gewichtsprozent bis 100 Gewichtsprozent aller enthaltenen Partikel enthält und einen gewichtsdurchschnittlichen Partikeldurchmesser (D50) von mindestens 300 $\mu$m und weniger als 500 $\mu$m aufweist und eine logarithmische Standardabweichung ($\sigma\zeta$) der Partikelverteilung von 0,20 bis 0,45 aufweist.

10. Verfahren nach einem der Ansprüche 1 bis 9, wobei der eluierte lösliche Inhalt des wasserabsorbierenden Mittels mindestens 0,1 Gewichtsteile und weniger als 30 Gewichtsteile enthält, basierend auf 100 Gewichtsteilen des wasserabsorbierenden Harzes.

11. Verfahren nach einem der Ansprüche 1 bis 10, wobei das wasserabsorbierende Harz erhalten wird, indem ein Monomer polymerisiert wird, das Acrylsäure (Salz) als einen Hauptbestandteil enthält.

**Revendications**

1. Procédé de fabrication d'un élément absorbant destiné à être utilisé dans des articles absorbants, ledit élément absorbant comprenant un agent absorbant fabriqué par le biais d'un procédé comprenant les étapes consistant à :

a) mélanger une résine absorbant l'eau avec de l'eau, et un auxiliaire de mélange sans ajouter d'initiateur de polymérisation radicalaire soluble dans l'eau et un monomère insaturé éthylénique et
b) irradier le mélange résultant avec des rayons ultraviolet en poursuivant l'agitation afin de conserver un état de mélange,

étape dans laquelle le temps d'exposition aux rayons est supérieur à 0,1 minute et inférieur à 60 minutes, la solution aqueuse ayant, dans ledit procédé, une tension superficielle dans la plage allant de 0,0004 N/cm à 0,00075 N/cm (de 40 dynes/cm à 75 dynes/cm), et l'absorbance de la solution saline physiologique sous une pression de 2,07 kPa de l'agent absorbant l'eau étant d'au moins 15 g/g et inférieur à 50 g/g et augmentant d'au moins 1 g/g par rapport à celui de la résine absorbant l'eau.

2. Procédé selon la revendication 1, la quantité d'eau à mélanger dans ledit procédé étant d'au moins 1 partie en poids et inférieure à 50 parties en poids en se basant sur les 100 parties en poids de la résine absorbant l'eau.

3. Procédé selon la revendication 1 ou la revendication 2, l'auxiliaire de mélange dans ledit procédé étant au moins un composé choisi dans le groupe comprenant des tensioactifs, un polymère soluble dans l'eau, des solvants organiques hydrophiles, des composés inorganiques solubles dans l'eau, des sels d'acides inorganiques, et des sels d'acides organiques.

4. Procédé selon l'une quelconque des revendications 1 à 3, l'auxiliaire de mélange dans ledit procédé étant ajouté en une quantité au moins égale à 0,01 parties en poids et inférieure à 50 parties en poids, en se basant sur les 100 parties en poids de la résine absorbant l'eau.

5. Procédé selon l'une quelconque des revendications 1 à 4, l'auxiliaire de mélange dans ledit procédé étant mélangé sous la forme d'une solution aqueuse avec la résine absorbant l'eau.

6. Procédé selon l'une quelconque des revendications 3 à 5, le tensioactif dans ledit procédé étant un éther d'alkyle du polyoxyéthylène.

7. Procédé selon l'une quelconque des revendications 3 à 5, le tensioactif dans ledit procédé étant le polyéthylène glycol.

8. Procédé selon l'une quelconque des revendications 3 à 5, le composé inorganique soluble dans l'eau dans ledit procédé étant un sel métallique soluble dans l'eau.

9. Procédé selon l'une quelconque des revendications 1 à 8, dans lequel la résine absorbant l'eau contient des particules ayant un diamètre particulaire d'au moins 150 $\mu$m et inférieur à 850 $\mu$m avec un rapport en poids de 90 % par rapport aux 100 % en poids de l'ensemble des particules à l'intérieur de celle-ci et a un diamètre particulaire moyen en poids (D50) d'au moins 300 $\mu$m et inférieur à 500 $\mu$m et un écart-type logarithmique ($\sigma\zeta$) de la distribution particulaire allant de 0,20 à 0,45.

10. Procédé selon l'une quelconque des revendications 1 à 9, dans lequel la teneur en agent absorbant l'eau soluble élué est d'au moins 0,1 partie en poids et inférieure à 30 parties en poids, en se basant sur les 100 parties en poids de la résine absorbant l'eau.

11. Procédé selon l'une quelconque des revendications 1 à 10, dans lequel la résine absorbant l'eau est obtenue en polymérisant un monomère ayant comme composant principal un (sel d') acide acrylique.

[Fig. 1]

[Fig. 2]

[Fig. 3]

(A)

The diameter
of the axis
8mm

(B)

The length
of the wing
30mm

The width
of the wing
10mm

30°

The thickness
of the wing
1mm

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- US 4910250 A
- JP 2004099789 A
- WO 2004031253 A
- US 4666983 A
- US 5422405 A
- US 4783510 A
- JP 2005097585 A
- JP 63260907 A
- US 4093776 A
- JP 4367323 B
- JP 4446261 B
- JP 4683274 B
- JP 5244735 B
- US 4625001 A
- JP 4873299 B
- JP 4286082 B
- JP 4973632 B
- JP 4985518 B
- JP 5124416 B
- JP 5250640 B
- JP 5264495 B
- JP 5145906 B
- JP 5380808 B
- EP 0811636 A
- EP 0955086 A
- EP 0922717 A
- US 5137537 A
- US 5147345 A
- US 5342338 A
- US 5260345 A
- US 5387207 A
- US 5397316 A
- US 5625222 A
- JP 2005270761 A

## Non-patent literature cited in the description

- *J. Phys. Chem.,* 1975, vol. 79, 2693
- *J. Photochem. Photobiol., A,* 1988, vol. 44, 243